Europäisches Patentamt
European Patent Office
Office européen des brevets

(19)

(11) Publication number: **0 309 422 A2**

(12) # EUROPEAN PATENT APPLICATION

(21) Application number: **88830374.0**

(22) Date of filing: **19.09.88**

(51) Int. Cl.[4]: **C 07 D 471/04**
C 07 D 223/26, C 07 D 223/18, C 07 D 243/38, C 07 D 495/04, C 07 D 403/06, C 07 D 401/06, C 07 D 451/00, C 07 D 519/00, C 07 D 405/06, C 07 D 403/12

(30) Priority: **21.09.87 IT 2197887**

(43) Date of publication of application:
**29.03.89 Bulletin 89/13**

(84) Designated Contracting States:
**AT BE CH DE ES FR GB GR IT LI LU NL SE**

(71) Applicant: **ISTITUTO DE ANGELI S.p.A.**
**Via Serio, 15**
**I-20139 Milano (IT)**

(72) Inventor: **Turconi, Marco**
**Via Gramsci, 20**
**Voghera (Pavia) (IT)**

**Donetti, Arturo**
**Viale Romagna, 4**
**Milano (IT)**

**Cereda, Enzo**
**Via Romagnolo, 2A**
**Tortona (Alessandria) (IT)**

**Quintero, Myrna Gil**
**Via Lamarmora, 3**
**Milano (IT)**

**Schiavi, Giovanni Battista**
**Via Montello, 7**
**Asola (Mantova) (IT)**

**Micheletti, Rosamaria**
**Via Borgospesso, 25**
**Milano (IT)**

(74) Representative: **Aimi, Luciano et al**
**c/o Società Italiana Brevetti S.p.A. Via Carducci 8**
**I-20123 Milano (IT)**

(54) **New amidino tricycle derivatives.**

(57) New pharmacologically active amidino tricycle derivatives as muscarinic receptor blocking agents, useful for the treatment of gastrointestinal disorders of the following formula:

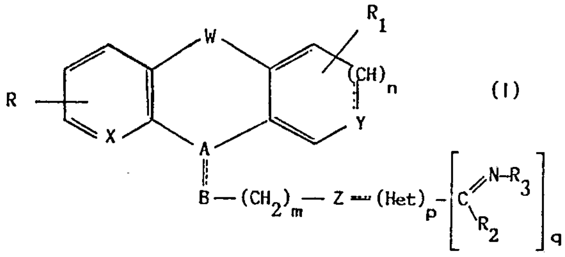

(I)

wherein
R represents a hydrogen atom or a halogen atom
X represents nitrogen or -CH-group
W represents a -NH-CO-, -CH=CH-, $-CH_2-CH_2-$ group, oxygen or sulfur
$R_1$ represents a hydrogen atom or a $C_{1-4}$ alkyl group
n is 0 or 1
Y represents sulfur or a -CH- group
A represents carbon or nitrogen
B represents a -CH- group (provided that A is different from nitrogen), -COO-, -CO-, or $-CH_2-$ group
m is 0 or an integer from 1 to 3
Z represents -NH-, -CO-, -COO- or -CH- group, or it is absent
p is 0 or 1
Het represents piperazinyl, homopiperazinyl, piperidinyl, tropyl or tetrahydropirimidinyl group, each group being optionally substituted by a $C_{1-4}$ alkyl group or an amino group
q is 0 or 1
$R_2$ represents a hydrogen atom, a $C_{1-4}$ alkyl group or an amino group optionally substituted by a linear or branched $C_{1-4}$ alkyl group or phenyl group

EP 0 309 422 A2

$R_3$ represents a linear or branched $C_{1-8}$alkyl group or a hydrogen atom (provided that the bond between Het and the group

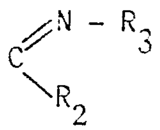

is a carbon- carbon bond or A=C and B=CH), or
$R_2$ and $R_3$ may join together to form a heterocyclic 5-membered ring, tautomers thereof and acid addition salts of the aforesaid compounds.

The process for the preparation of the compounds of formula (I) as well as pharmaceuticals compositions containing them are also described.

**Description**

## NEW AMIDINO TRICYCLE DERIVATIVES

The present invention relates to novel pharmacologically active amidino tricycle derivatives, to the process for their preparation and to the pharmaceutical compositions containing them. The new compounds are muscarinic receptor blocking agents useful for the treatment of gastrointestinal disorders.

It is known that the administration of muscarinic receptor blocking agents gives rise to a number of pharmacological effects like decreased gastrointestinal motility, inhibition of acid secretion, dry mouth, mydriasis, urinary incontinence, decreased sweating, tachycardia. Furthermore, antimuscarinic agents with tertiary amine structure may give rise to central effects owing to their penetration across blood-brain barrier. The lack of selectivity among these actions makes it difficult to address therapy in one specific indication and this prompted chemical modification of these agents. One of these modifications consists in quaternization of the tertiary amine function to prevent penetration into the brain. The quaternary drugs lack prominent central actions and additionally show a selective greater effect on the gastrointestinal tract, while displaying a minor incidence of side-effects. However their major drawback is the poor and unreliable absorption for oral administration unexploitable for therapeutical purposes. We have now synthetized, and this is the object of the present invention, a new class of amidino tricycle derivatives endowed with a strong antimuscarinic activity which shows a further enhanced activity on the gastrointestinal tract associated with a lack of central and peripheral effects such as mydriasis, tachycardia and dry mouth. Moreover the new tricycle derivatives are potentially useful as therapeutically active agents in the management of gastrointestinal motility disorders, such as spastic conditions of the gut, functional diarrhea, constipation, irritable bowel syndrome, cardiospasm, pylorospasm, gastro-oesophageal reflux, peptic ulcer disease, spasm of the urinary and biliary tracts and urinary incontinence. The compounds, object of the present invention, have the general formula (1)

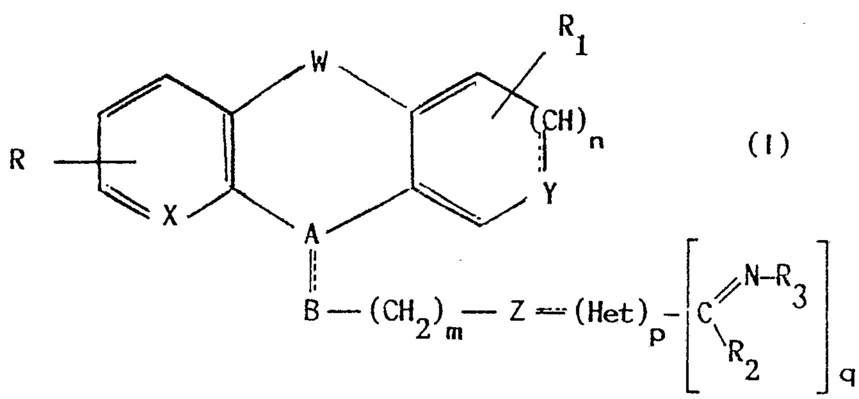

(I)

wherein

R represents a hydrogen atom or a halogen atom

X represents nitrogen or -CH-group

W represents a -NH-CO-, -CH=CH-, $-CH_2-CH_2-$ group, oxygen or sulfur

$R_1$ represents a hydrogen atom or a $C_{1-4}$ alkyl group

n is 0 or 1

Y represents sulfur or a -CH- group

A represents carbon or nitrogen

B represents a -CH- group (provided that A is different from nitrogen), -COO-, -CO-, or $-CH_2-$ group

m is 0 or an integer from 1 to 3

Z represents -NH-, -CO-, -COO- or -CH- group, or it is absent

p is 0 or 1

Het represents piperazinyl, homopiperazinyl, piperidinyl, tropyl or tetrahydropirimidinyl group, each group being optionally substituted by a $C_{1-4}$ alkyl group or an amino group

q is 0 or 1

$R_2$ represents a hydrogen atom, a $C_{1-4}$ alkyl group or an amino group optionally substituted by a linear or branched $C_{1-4}$ alkyl group or phenyl group

$R_3$ represents a linear or branched $C_{1-8}$alkyl group or a hydrogen atom (provided that the bond between Het and the group

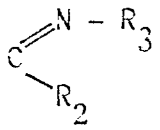

is a carbon - carbon bond or A=C and B=CH) or
$R_2$ and $R_3$ may join together to form a heterocyclic 5-membered ring, tautomers thereof and acid addition salts of the aforesaid compounds.

For pharmaceutical use the compounds (I) are used as such or under the form of tautomers or of physiologically compatible acid addition salts. The term "acid addition salts" includes salts with inorganic or organic acids. The physiologically compatible acids used for the salification include, for example, salts formed with maleic, citric, hydrochloric, tartaric, hydrobromic, tumaric,nitric, sulphuric, methanesulphonic or idroiodic acid. Although the double bond of the amidine groupments is indicated in the general formula (I) as present in a particular position, other tautomeric forms are also possible. The present invention includes therefore such tautomeric forms as regards both the compounds and the manufacturing processes.

Some compounds of formula (I), according to the present invention, contain one or two asymmetric carbon atoms. The compounds may therefore occur as enantiomers of (+) and (-) type, as diastereoisomers or mixture of them. The present invention includes therefore both the individual isomers and the mixture thereof.

It has to be understood that, when mixtures of optical isomers are present, they may be separated according to the classic resolution methods based on their different physico-chemical properties, e.g. by the fractional crystallization of their acid addition salts with a suitable optically active acid or by the chromatographic separation with a suitable mixture of solvents.

In the present invention the term "lower alkyl" means that the group is a straight or branched alkyl and it has preferably 1 to 4 carbon atoms. The term "halogen" means fluorine, bromine, chlorine and iodine.

The compounds of general formula (I) of the present invention may, for example, be prepared by the following processes well known in their general lines to any skilled chemist.

a) Compounds of general formula (I), wherein R, $R_1$, $R_3$, X, Y, W, A, B, Z, Het, n, m, p, are as hereinbefore defined, q is 1 and $R_2$ is an amino group optionally substituted by a linear or branched $C_{1-4}$ alkyl group or a phenyl group, may be prepared by reacting a compound of general formula (II)

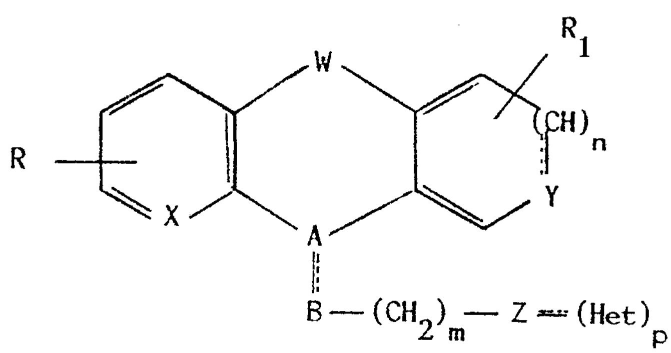

(II)

(provided that Het is different from tetrahydropyrimidine and contains at least one secondary amino function when p is 1) wherein R, $R_1$, X, W, Y, A, B, Z, Het, m, n and p are as above defined in the form of its acid addition salt with a mineral acid of formula H M such as hydrochloric, hydrobromic, hydroiodic, sulphuric, nitric, tetrafluoboric acid, preferably hydrochloric acid with cyanamide in the absence of any solvent. The reaction is conventionally carried out at a temperature ranging from 70° to 160°C, prefarably at 100°C. If desired the reaction can be carried out in the presence of a protic solvent such as methanol, ethanol, water or a mixture of them at a temperature between 50° and 100°C preferably at the boiling point of the solvent. The same compounds (I) may be prepared by reacting a compound of formula (II) with a reactive compound of general formula (III)

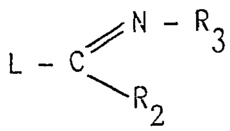
(III)

where L is a suitable leaving group selected from 3,5-dimethyl-pyrazol-1-yl, lower thioalkyl, preferably thiomethyl or sulphonyl; the compounds of formula (III) if necessary may be reacted in the form of their addition salts with a mineral acid of formula H M as hereinbefore defined. The reaction is carried out in polar solvents such as methanol, ethanol, acetonitrile, acetone, water or a mixture of them at a temperature ranging from 20° to 100°C preferably at the reflux temperature of the selected solvent. The same compounds(I), if desired, may be obtained by reducing a nitroguanidine derivative of general formula (IV)

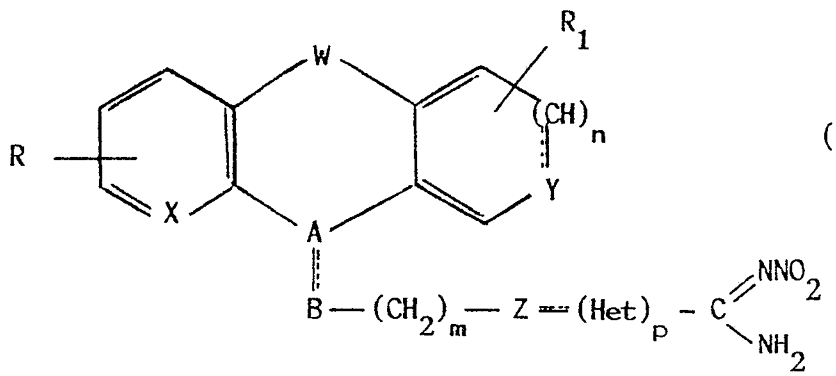
(IV)

(provided that the nitroguanyl moiety is linked to the secondary nitrogen atom of Het when p is 1) wherein R, $R_1$, X, Y, W, A, B, Z, Het, n, m, p are as hereinbefore defined with hydrogen or a hydrogen donor such as formic acid, acetic acid, ammonium formate, cyclohexene, cyclohexadiene, preferably formic acid in the presence of a suitable catalyst preferably Pd/C or Pd black, in the presence or in the absence of a suitable solvent such as formic acid, water, methanol, ethanol, or mixtures of them, preferably formic acid. The same reduction may be conveniently carried out with reducing agents such as Titanium (III) chloride in hydroalcoholic solvents or Tin (II) chloride in diluted formic acid. The reactions are performed at a temperature ranging from 10° to 100°C, preferably at 40°C. Compounds of formula (IV), used as starting material in the above processes, may be prepared by reacting a compound of formula (II) with a reactive intermediate of general formula (V)

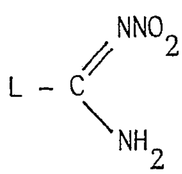
(V)

where L is as hereinbefore defined. The reaction is carried out in solvents such as methanol, ethanol, chloroform,methylenedichloride or mixtures of them, preferably in a 1:1 mixture of methanol and methylenedichloride at a temperature ranging from 10° to 80°C, preferably at room temperature.
In an additional option the same compounds (I) may be prepared by reacting a compound of general formula (VI)

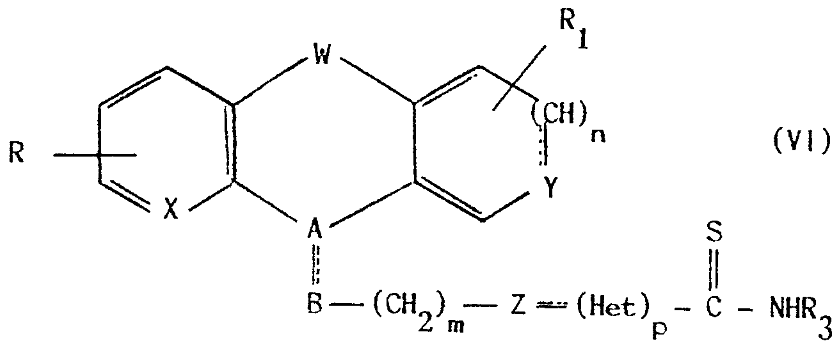

(VI)

where R, $R_1$, $R_3$, X, Y, W, A, B, Z, Het, n, m, p are as hereinbefore defined (provided that the thiocarbamoyl moiety is linked to the secondary nitrogen atom of the Het when p is 1) with an alkylating agent such as methyliodide or dimethylsulphate and then reacting in situ the corresponding isothiouronium intermediate with an amine of formula $R_2$ - H in which $R_2$ is as hereinbefore defined. The reaction is carried out in a polar solvent such as methanol, ethanol, acetonitrile, acetone, water or a mixture of them at a temperature ranging from 20° to 100°C, preferably at the reflux temperature of the selected solvent. The thiourea derivatives of general formula (VI) used as starting materials in the above described process may be prepared by reacting a compound of formula (II) or its hydrochloric acid addition salt with an isothiocyanate of general formula (VII)

$R_3 - N = C = S$ (VII)

where $R_3$ is as hereinbefore defined, or with ammonium thiocyanate. The reaction is carried out in solvents such as water, methanol, ethanol, tetrahydrofurane, acetone, preferably tetrahydrofurane or water or without solvents at a temperature ranging from 0° to 200°C, preferably at room temperature, at 100°C or at the melting point of the reagents mixture.

b) Compounds of general formula (I) wherein R, $R_1$, $R_3$, X, Y, W, A, B, Z, Het, n, m, p are as hereinbefore defined, q is 1 and $R_2$ is a hydrogen atom or a $C_{1-4}$ alkyl group, may be prepared by reacting a compound of general formula (II) (provided that Het is not a tetrahydropyrimidine and contains at least one secondary amino function) with an addition salt of a compound of general formula (VIII)

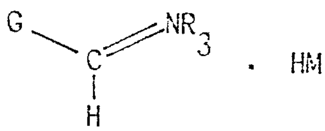

(VIII)

wherein $R_3$ and HM are as above defined and G is a suitable leaving group such as halogen, lower alkoxy, phenoxy, dichloro phosphoryl, preferably ethoxy or methoxy, when $R_2$ is a hydrogen atom; or with a compound of general formula (IX)

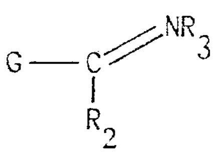

(IX)

in which $R_2$, $R_3$ and G are as above defined, when $R_2$ is a $C_{1-4}$ alkyl group. The compounds of formula (II) and (VIII) or (IX) may be conveniently used either as free bases or as addition salts with mineral acids of formula HM as above defined. The reaction is conveniently carried out in a polar solvent such as methanol, ethanol, acetonitrile, acetone, ethyl acetate or mixtures of them. The reaction temperature is generally kept between 10° and 70°C, preferably at room temperature.

If desired when $R_2$ represents a hydrogen atom, the same compounds may be also conveniently obtained by desulphurizing a thiourea of general formula (VI) with Raney-Nickel or $H_2O_2$ in an appropriate solvent

selected from dichloromethane, chloroform, ethanol, water or a mixture of them. The process is carried out at a temperature between 10° and 70°C, preferably at room temperature.

c) Compounds of general formula (I), wherein R, $R_1$, $R_3$, X, Y, W, A, B, Z, Het, n, m, p are as hereinbefore defined, q is 1 and $R_2$ is an amino group optionally substituted by a linear or branched $C_{1-4}$ alkyl group or phenyl group, may be prepared by reacting a cyano derivative of general formula (X)

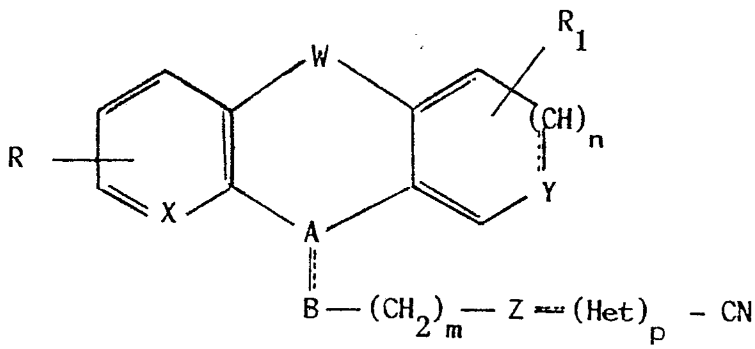

(X)

in which R, $R_1$, X, Y, W, A, B, Z, Het, n, m, p are as hereinbefore defined (provided that the cyano moiety is linked to a carbon atom of Het when p is 1 and Het is different from tetra hydropirimidine) with a substituted or unsubstituted ammonium salt of formula (XI)

$R_2H$ . HM (XI)

where $R_2$ and HM are as hereinbefore defined, or with a substituted or unsubstituted thiourea of formula (XII)

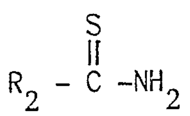

(XII)

The reaction is simply carried out by mixing the two reagents and heating the mass above its melting point at a temperature between 50° and 200°C, preferably between 100° and 200°C. Optionally the reaction between a compound of formula (X) and a compound of formula (XI) may be carried out in the presence of a Lewis acid such as aluminium chloride, zinc chloride, iron (III) chloride, tin (IV) chloride, triphenyltinchloride, preferably aluminium chloride. The reaction is carried out in an inert, high boiling, halogenated hydrocarbon such as chlorobenzene, tetrachloroethane or without any solvent as a molten mass. The reaction temperature ranges from 50° to 200°C, preferably at the boiling point of the selected solvent. If desired, the same compounds may be obtained by reacting an imidate or an imidoyl derivative of general formula (XIII)

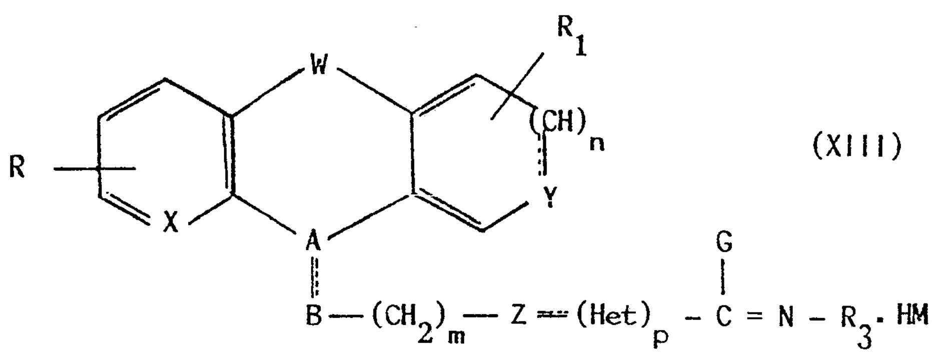

(XIII)

wherein R, $R_1$, $R_3$, X, Y, W, A, B, Z, Het, n, m, p, HM, and G are as hereinbefore defined with a compound of formula (XI) as a free base. The reaction is conveniently carried out in a solvent such as methanol, ethanol, acetonitrile, acetone, or mixtures of them at a temperature ranging from 0° to 80°C preferably at room temperature.

d) Compounds of general formula (I), wherein R, $R_1$, X, Y, W, A, B, are as hereinbefore defined, m is 2, Het is a N-linked tetrahydropyrimidinyl group optionally substituted by a $C_{1-4}$ alkyl group or an amino group, Z is absent and q is 0, may be prepared by reacting a compound of general formula (XIV)

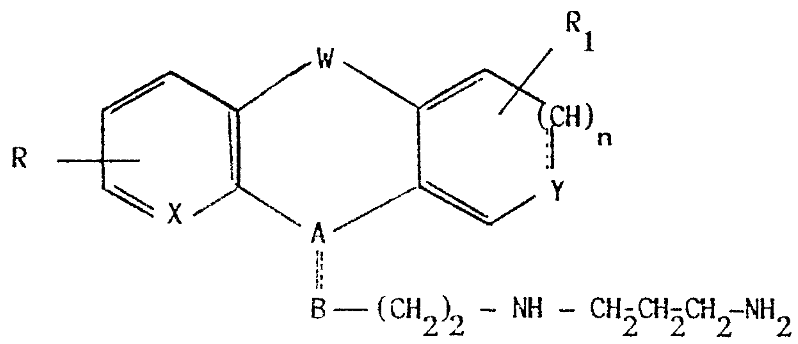

(XIV)

wherein R, $R_1$, X, Y, W, A, B, n, are as hereinbefore defined, with a compound of general formula (XV)

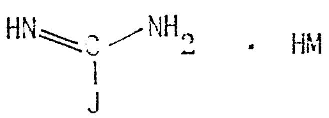

(XV)

wherein J is a hydrogen atom, a $C_{1-4}$ alkyl or an amino group in the form of its addition salt with a mineral acid of formula HM. The process can be carried out in a suitable solvent such as methanol, ethanol, acetonitrile, acetone, preferably ethanol, at a temperature ranging from 0° to 80°C, preferably at the boiling point of the selected solvent.

In an additional option, the same compounds, wherein Het is an N-linked tetrahydropyrimidinyl group substituted by an amino group, can also be obtained by reducing a compound of general formula (XVI)

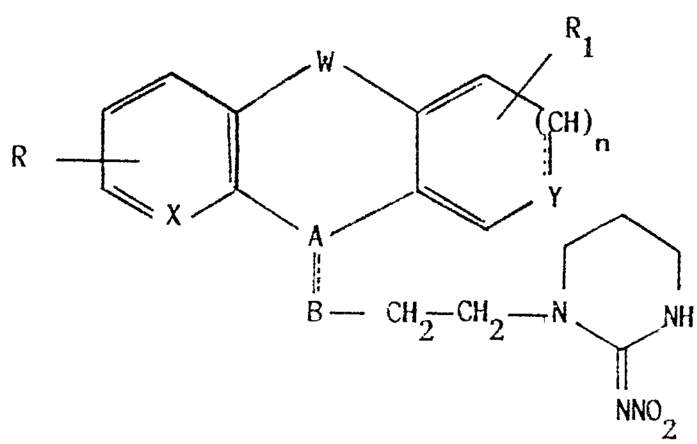

(XVI)

in which R, $R_1$, X, Y, W, A, B, n are as hereinbefore defined, with hydrogen or a hydrogen donor such as formic acid, acetic acid, ammonium formate, cyclohexene, cyclohexadiene, preferably formic acid in the presence of a suitable catalyst preferably Pd/C or Pd black in the presence or in the absence of a suitable solvent such as formic acid, water, methanol, ethanol or mixtures of them, preferably formic acid. The process is performed at a temperature ranging from 10° to 200°C,preferably at 40°C. Compounds of formula (XVI) may be conveniently prepared by reacting a diamino compound of formula (XIV) with a compound of formula (V). The process is performed in an inert solvent such as methanol, ethanol, chloroform, methylene dichloride or mixtures of them preferably in a 1:1 mixture of methanol and methylene dichloride at a temperature ranging from 10°C to 80°C, preferably at room temperature.

The compounds of general formula (I), prepared according to the processes as above described may

optionally be converted with inorganic or organic acids into the corresponding physiologically compatible acid addition salts, for example, by conventional methods such as by reacting the compounds as bases with a solution of the corresponding acid in a suitable solvent. Particularly preferred acids include for example hydrochloric, hydrobromic,hydroiodic, sulphuric and methanesulphonic acid.

As already mentioned hereinbefore, the new compounds of formula (I), according to the present invention, have interesting pharmacological properties owing to their ability to antagonize the physiological muscarinic effects in warm blooded animals. Therefore the new compounds are advisable in the prevention or in the treatment of motility disorders wherein muscarinic receptors are involved.

The following experimental data show that the compounds according to the invention have favourable characteristics in this respect.

**PHARMACOLOGY**

ANTIMUSCARINIC ACTIVITY (in vitro binding studies)

Antimuscarinic activity was examined by studying the displacement of $^3$H-pirenzepine from cerebral cortex homogenate according to the procedure reported below:

The cerebral cortex donors were male CD-COOBBS rats, 220-250 g body weight. The homogenization process was carried out in a Potter-Evelhjem apparatus in the presence of $Na^+/Mg^{++}$ HEPES buffer; pH 7.4 (100 mM NaCl, 10 mM $MgCl_2$, 20 mM HEPES), by filtering the suspension through two layers of cheesecloth.

Binding curves for the under study compounds were derived indirectly from competition experiments against 0.5 nM $^3$H-pirenzepine labelling the muscarinic receptors of the cerebral cortex. 1 ml of the homogenate was incubated for 45 min at 30° C in the presence of a marker ligand and different concentrations of the cold ligand, conditions under which equilibrium was reached as determined by appropriate association experiments. The incubation was terminated by centrifugation (12,000 rpm for 3 min) at room temperature using an Eppendorf microcentrifuge. The resultant pellet was washed twice with 1.5 ml saline to remove the free radioactivity and it was allowed to drain for some hours. The tips of the tubes containing the pellet were cut off and 200 µl of tissue solubilizer (Lumasolve, Lumac) were added and left to stand overnight. Radioactivity was then counted after addition of 4 ml of liquid scintillation mixture (Dimilume/Toluene 1:10 v:v, Packard)

Assays were carried out in triplicate or quadruplicate and the non-specific binding was defined as the radioactivity bound or entrapped in the pellet when the incubation medium contained 1 µM atropine sulphate. Non-specific binding avaraged less than 30%. $K_D$ values (dissociation constants) were obtained by non-linear regression analysis on the basis of a one binding site model with TOPFIT-pharmacokinetic programme package (G. HEINZEL, "Pharmacokinetics during drug development: data analysis and evaluation techniques" Eds. G. BOLZER and J.M. VAN ROSSUM; p. 207, G. Fisher, New York, 1982) after correction for the radioligand occupancy shift according to the equation: $K_D = IC_{50}/1 + {}^*C/{}^*K_D$, where *C and $^*K_D$ represent the concentration and the dissociation constant of the radioligand used, respectively.

The following table I shows the obtained results:

TABLE I

Antimuscarinic effect. Dissociation constants ($K_D$) for $^3$H-pirenzepine binding

| Compound | $K_D$ (nM) |
|---|---|
| 3 | 6 |
| 19 | 4 |
| 20 | 3.2 |
| 26 | 9 |
| 31 | 7.7 |
| 60 | 2.7 |
| 61 | 5 |
| 62 | 8 |
| 72 | 4 |
| 78 | 5 |
| 81 | 10 |
| 82 | 10 |
| 83 | 7 |
| 84 | 2.5 |
| 85 | 5 |

According to a further feature of the present invention there are provided pharmaceutical compositions comprising as active ingredient at least one compound of formula (I), as hereinbefore defined, or a physiologically compatible acid addition salt thereof in association with a pharmaceutical carrier or excipient. For pharmaceutical administration the compounds of general formula (I) and their physiologically compatible acid addition salts may be incorporated into the conventional pharmaceutical preparations in either solid or liquid form. The compositions may, for example, be presented in a form suitable for oral, rectal or parenteral administration. Preferred forms include, for example, capsules, tablets, coated tablets, freeze-dried vials, suppositories and oral drops.

The active ingredient may be incorporated in excipients or carrier conventionally used in pharmaceutical compositions such as, for example, talc, gum arabic, lactose, gelatine, magnesium stearate, corn starch, aqueous or non-aqueous vehicles, polyvinylpirrolidone, mannitol, semisynthetic glicerides of fatty acids, sorbitol, propylene glycol, citric acid, sodium citrate. The compositions are advantageously formulated at dosage units, each dosage unit being adapted to supply a single dose of the active ingredient. Each dosage unit may conveniently contain from 5 mg to 500 mg and preferably from 10 mg to 100 mg.

The following examples illustrate some of the new compounds according to the present invention; these examples are not to be in any way considered limitative of the scope of the invention itself:

## Example 1

### 3-chloro-5,11-dihydro-11-[2-(1-piperazinyl)acetyl]-6H-pyrido[2,3-b] [1,4]-benzodiazepin-6-one

a) A mixture of 5,11-dihydro-6H-pyrido[2,3-b] [1,4]-benzodiazepin-6-one (5.0 g), glacial acetic acid (15 ml) and 120 vol. hydrogen peroxide (15 ml) was heated to 100°C under stirring. The temperature was maintained for an hour then the reaction mixture was left overnight. Crude 5,11-dihydro-6H-pyrido-[2,3-b] [1,4]-benzodiazepin-6-one -1-oxide was collected by filtration. Yield 2.1 g, M.p. 230°-232°C.

b) A mixture of 5,11-dihydro-6H-pyrido-[2,3-b] [1,4]-benzodiazepin-6--one-1-oxide (21.9 g) and phosphorous oxychloride (200 ml) was heated to reflux for 90 min. The cooled reaction mixture was evaporated to dryness under vacuum then taken up with ice/water. The solid 3-chloro-5,11-dihydro-6H-pyrido[2,3-b] [1,4]-benzodiazepin-6-one (19,7 g) was collected by filtration. M.p. 220°-223°C.

c) To a suspension of 3-chloro-5,11-dihydro-6H-pyrido-[2,3-b] [1,4]-benzodiazepin-6-one (19.5 g) in dioxane (200 ml) triethylamine (8.0 g) was added. The suspension was heated to reflux while chloroacetylchloride (18.0 g) was dropped in. Reflux was kept for four hours then the solid which separated after cooloing was discarded. The reaction mixture was taken to dryness and crude 3-chloro-11-(2-chloroacetyl)-5,11-dihydro-6H-pyrido[2,3-b] [1,4]-benzodiazepin-6-one was triturated with diethyl ether and collected by filtration. Yield 19.8 g, M.p. 203°-206°C. Analogously was prepared: 5-(2-chloroacetyl)-5H-dibenz[b,f]azepine. M.p. 188°-190°C.

d) To a solution of anhydrous piperazine (11.7 g) in tetrahydrofurane (240 ml) 3-chloro-11-(2-chloroacetyl)-5,11-dihydro-6H-pyrido[2,3-b] [1,4]-benzodiazepin-6-one (8.8 g) dissolved in the same solvent (40 ml) was added. The reaction mixture was stirred at room temperature overnight, then filtered. The solid was thoroughly washed with water and desiccated. The mother liquors were concentrated to dryness and the residue was triturated with water and collected by filtration. The two solids were crystallized from ethanol to give pure title compound (3.0 g) M.p. 240°-242°C.

The following piperazine derivatives were analogously prepared starting from the appropriate chloroacetyl compound:

5-[2-(1-piperazinyl)-acetyl]-5H-dibenz[b,f]azepine. M.p. 165°-167°C.

10,11-dihydro-5-[2-(1-piperazinyl)-acetyl]-5H-dibenz[b,f]azepine. M.p. 163°-164°C.

5,10-dihydro-5-[2-(1-piperazinyl)-acetyl]11H-dibenzo[b,e] [1,4]diazepin-11-one. M.p. 210°-211°C.

5,11-dihydro-11-[2-(1-piperazinyl)-acetyl]-6H-pyrido[2,3-b] [1,4]benzodiazepin-6-one. M.p. 249°-250°C.

4,9-dihydro-4-[2-(1-piperazinyl)-acetyl]-10H-thieno[3,4-b] [1,5]benzodiazepin-10-one. M. p. 181°-182°C.

## Example 2

### 5,10-dihydro-4-methyl-5-[2-(1-piperazinyl)acetyl]-11H-dibenzo[b,e] [1,4]-diazepin-11-one.

a) A mixture of 2-bromonitrobenzene (28.51 g),3-methylanthranilic acid (32 g), potassium carbonate (29.25 g) and copper powder (1 g) in isoamyl alcohol (400 ml) was heated to reflux for 2 hours. The cooled reaction mixture was evaporated to dryness and the residue was triturated with water. The solids were discarded while the mother liquors were acidified to pH 2. Solid 3-methyl-N-(2-nitrophenyl)anthranilic acid separated and was recovered by filtration. Yield 31.9 g. M.p. 182°-183°C.

b) A solution of 3-methyl-N-(2-nitrophenyl)-anthranilic acid (31.0 g) in ethanol (700 ml) was hydrogenated at room temperature and atm. pressure in the presence of 10% Pd/C (3.1 g). Sufficiently pure 3-methyl-N-(2-aminophenyl)-anthranilic acid (19.9 g) was obtained after removal of the catalyst. M.p. 210°C-212°C.

c) A solution of 3-methyl-N-(2-aminophenyl)-anthranilic acid (19.0 g) in xilene (400 ml) was heated in the presence of a Dean-Stark separator for the ozeotropic removal of water. Heating was kept for 18

hours then, upon cooling, the reaction mixture separated pure 5,10-dihydro-4-methyl-11H-dibenzo[b,e] [1,4]-diazepin-11-one. Yield 15.2 g, M.p. 212°-213°C.

d) To a solution of 5,10-dihydro-4-methyl-11H-dibenzo[b,e] [1,4]-diazepin-11-one (15 g) in hot xilene chloroacetylchloride (8.9 g) was added dropwise in 15 min. Heating and stirring at 100°C was continued for 3 hours then, upon cooling, 5-(2-chloroacetyl)-5,10-dihydro-4-methyl-11H-dibenzo[b,e] [1,4]-diazepin-11-one crystallized out. Yield 18.8 g, M.p. 238°-240°C.

The following (2-chloroacetyl) derivative was also prepared utilizing the procedure above described, starting from the appropriate tricyclic compound.

6-chloro-5,10-dihydro-5-(2-chloroacetyl)-11H-dibenzo[b,e] [1,4]-diazepin-11-one. M.p. 243°-244°C.

Analogously was also prepared:

5-(4-chlorobutynyl)-5,10-dihydro-11H-dibenzo[b,e] [1,4]-diazepin-11-one. M.p. 230°-232°C.

e) The title compound was prepared according to the procedure described in Example 1 d) M.p. 198°-199°C. Similarly was also prepared:

5,10-dihydro-6-chloro-5-[2-(1-piperazinyl)-acetyl]-11H-dibenzo [b,e] [1,4]-diazepin-11-one. M.p. 200°-202°C.

Example 3

5,6-dihydro-11-[2-(1-piperazinyl)ethyliden)]-11H-dibenz[b,e]azepin-6-one.

a) 5,6-dihydro-11H-dibenz[b,e]azepin-6,11-dione (20.0 g) was portion wise added to a solution of 1.3 M vinylmagnesiumbromide in tetrahydrofurane (140 ml) while stirring at 0°-5°C under a nitrogen atmosphere. After the initial exothermic reaction had subsided the temperature was raised to 25°C, stirring was continued for 2 hours then the reaction mixture was allowed to stand overnight. After quenching with saturated ammonium chloride solution the phases were separated and the organic one was evaporated to dryness. After crystallization from ethylacetate there were obtained 16.7 g of 5,6-dihydro-11-hydroxy-11-vinyl-11H-dibenz[b,e]azepin-6-one. M.p. 185°-187°C.

b) To a solution of 5,6-dihydro-11-hydroxy-11-vinyl-11H-dibenz[b,e] azepin-6-one (20.0 g) in methylene chloride (500 ml) cooled at -10°C was added phosphorous pentachloride (20,7 g) dissolved in the same solvent (500 ml). The addition required 20 min then the temperature was allowed to come to 25°C. Stirring was continued for two and a half hours then saturated sodium bicarbonate (1 lt) was added. The organic layer was separeted and dried. The raw material was purified by flash chromatography (eluent: methylene chloride/methanol 95:5) on silicagel. Pure 5,6-dihydro-11-(2 chloro ethyliden)-11H-dibenz[b,e]azepin-6-one (9.4 g) was recovered. M.p. 189°-191°C.

c) The title compound was obtained according to the procedure described in Example 1d). M.p. 122°-124°C.

Example 4

5,11-dihydro-11-[2-(1-homopiperazinyl)-acetyl]-6H-pyrido[2,3-b] [1,4]-benzodiazepin-6-one

Homopiperazine (6.96 g) was dissolved in tetrahydrofurane (250 ml) and to the resulting solution 11-(2-chloroacetyl)-5,11-dihydro-6H-pyrido[2,3-b] [1,4]-benzodiazepin-6-one (4.0 g) was added portionwise. The reaction mixture was stirred for 24 hrs at room temperature and then filtered from the insoluble material. The mother liquors were concentrated to dryness and pure title compound was obtained by flash chromatography technique (eluent methylene-chloride-methanol-32% $NH_4OH$ 80:20:2) on silicagel and crystallization from benzene. 2,7 g.M.p. 230°-232°C.

The following homopiperazine derivatives were prepared from the appropriate chloroacetyl compounds and homopiperazine according to the above described procedure:

5,10-dihydro-5-[2-(1-homopiperazinyl)-acetyl]-11H-dibenzo[b-e] [1,4]-diazepin-11-one. M.p. 190°-192°C.

4,9-dihydro-4-[2-(1-homopiperazinyl)-acetyl]-10H-thieno[3,4-b] [1,5]-benzodiazepin-10-one. M.p. 185°-187°C.

Example 5

5,10-dihydro-5-[2-(2,5-dimetyl-1-piperazinyl)-acetyl]-11H-dibenzo [b,e] [1,4]-diazepin-11-one;

To a solution of 2,5-dimethylpiperazine (mixture of cis and trans isomers) (4.26 g) in tetrahydrofurane a solution of 5-(2-chloroacetyl)-5,10-dihydro-11H-dibenzo[b,e] [1,4]-diazepin-11-one (1.5 g) in tetrahydrofurane (150 ml) was added dropwise under stirring. The reaction mixture was stirred at room temperature for further 24 hrs, then worked up as described before. 1.34 g. M.p. 140°-143°C.

The following 2,5-dimethylpiperazine derivatives were also prepared utilizing the above described procedure, starting from the appropriate chloroacetyl compound:

5,11-dihydro-11-[2-(2,5-dimethyl-1-piperazinyl)-acetyl]-6H- pyrido [2,3-b] [1,4]-benzodiazepin-6-one. M.p. 150°-155°C.

4,9-dihydro-4-[2-(2,5-dimethyl-1-piperazinyl)-acetyl]-10H-thieno [3,4-b] [1,5]-benzodiazepin-10-one. M.p. 130°-134°C.

Example 6

5-(2-bromoacetyl)-5,10-dihydro-11H-dibenzo[b,e] [1,4]-diazepin-11-one.

To a solution of 5,10-dihydro-11H-dibenzo[b,e] [1,4-diazepin-11-one (20.0 g) in hot xylene (200 ml, 100°C ) bromoacetylchloride (17.9 g) was added dropwise in 15 min. Heating was continud for 90 min then, upon cooling, the title compound was separated. Yield 28 g. M.p. 215°-216°C.

Example 7

11-(2-bromoacetyl)-5,11-dihydro-6H-pyrido[2,3-b] [1,4]-benzodiazepin-6-one.

To a suspension of 5,11-dihydro-6H-pyrido[2,3-b] [1,4]-benzodiaze-pin-6-one (28 g) in tetrahydrofurane (500 ml) triethylamine (14.7 g) was added and the whole was heated to reflux. Bromoacetylbromide (53.5 g) was added dropwise in 30 min and heating was continued for sixteen hours under stirring. Upon cooling the reaction mixture was filtered and the solids were discarded. The mother liquors were concentrated to dryness then pure title compound was crystallized from acetonitrile. Yield 10.2 g. M.p. 207°-208°C.

Example 8

4-(2-bromoacetyl)-4,9-dihydro-10H-thieno[3,4-b] [1,5]-benzodiazepin-10-one.

A suspension of 4,9-dihydro-10H-thieno[3,4-b] [1,5]-benzodiazepin-10-one (7.9 g) in dry dioxane (300 ml) was stirred at room temperature overnight in the presence of bromoacetylbromide (9.6 g). The reaction mixture was then concentrated to dryness and 3.05 g of the title compound were recovered after trituration of the residue with diethyl ether. M.p. 195°-197°C.

Example 9

5,10-dihydro-5-[2-(4-piperidinyl)-acetyl]-11H-dibenzo[b,e] [1,4]-diazepin-11-one.

a) A mixture of 5-(2-bromoacetyl)-5,10-dihydro-11H-dibenzo[b,e] [1,4]-diazepin-11-one (20 g) and triethylphosphite (20 g) was heated to 150°C until reaction took place. The temperature was kept for 30 min, then vacuum was applied to the flask containing the reaction mixture. The residue was dissolved in methylene-chloride and diluted with petroleum ether. Crystallization took place yielding 22.3 g of 5,10-dihydro-5-(2-diethylphosphonoacetyl)-11H-dibenzo[b,e] [1,4]-diazepin-11-one. M.p. 195°-196°C.
Analogously were prepared:
5,11-dihydro-11-(2-diethylphosphonoacetyl)-6H-pyrido[2,3-b] [1,4]-benzodiazepin -6-one. M.p. 210°-211°C.
4,9-dihydro-4-(2-diethylphosphonoacetyl)-10H-thieno[3,4-b] [1,5]-benzodiazepin-10-one. M.p. 180°-182°C.

b) 80% sodium hydride (4.86 g) in tetrahydrofurane (350 ml) were placed in a reaction flask under a nitrogen atmosphere. To the suspension were added in batches 35 g of 5,10-dihydro-5-(2-diethylphosphonoacetyl)-11H-dibenzo[b,e] [1,4]-diazepin-11-one and the resulting suspension was gently refluxed for two hours. At the same temperature 1-benziloxycarbonyl-4-piperidone (18.9 g) dissolved in tetrahydrofurane (150 ml) was added dropwise and then the reaction mixture was refluxed for additional 2 hrs. Saturated ammonium chloride solution was added and the aqueous layer was washed twice with ethyl acetate. After evaporation to dryness the organic phases left 30 g of a mixture of 5,10-dihydro5-[2-(1-benzyloxycarbonyl-4-piperidinylidene)-acetyl]-11H-dibenzo [b,e] [1,4]-diazepin-11-one and 5,10-dihydro-5-[2-(1-benzyloxycarbonyl-1,2,5,6-tetrahydro-4-pyridinyl)-acetyl]-11H-dibenzo[b,e] [1,4]diazepin-11-one.
TLC: (benzene-acetic acid - ethylacetate 9:0.5:1). Rf= 0.3 and 0.4 on Silicagel plates.
Analogously were prepared the following mixtures of isomers or the following single compound starting from the appropriate compounds: 5,11-dihydro-11-[2-(1-benzyloxycarbonyl-4-piperidinylidene)-acetyl]-6H-pyrido[2,3-b] [1,4]benzodiazepin-6-one and 5,11-dihydro-11-[2-(1-benzyloxycarbonyl-1,2,5,6-tetrahydro-4-pyridinyl)-acetyl]-6H--pyrido[2,3-b] [1,4]-benzodiazepin-6-one.
TLC : methylene chloride - methanol - 32% $NH_4OH$ 90:10:1)
Rf = 0.61 and 0.67 on Silicagel plates.

4,9-dihydro-4-[2-(1-benzyloxycarbonyl-4-piperidinylidene)-acetyl]-10H-thieno[3,4-b] [1,5]-benzodiazepin 10-one and 4,9-dihydro-4-[2-(1-benzyloxycarbonyl-1,2,5,6-tetrahydro-4-pyridinyl)-acetyl]-10H-thieno[3,4-b] [1.5]-benzodiazepin-10-one.
TLC: (methylene chloride - methanol - 32% $NH_4OH$ 90:10:1)
Rf = 0.65 and 0.70 on Silicagel plates.

5,10-dihydro-5-[2-(1-benzyl-3-piperidinylidene)-acetyl]-11H-dibenzo [b,e] [1,4]-diazepin-11-one.

M.p. 133°-135°C.
TLC: (methylene chloride - methanol - 32% $NH_4OH$ 90:10:1)
Rf = 0.68
Similarly starting from 8-vinyloxycarbonyl-8-azabicyclo[3.2.1]octan-3-one (see Example 12 a) it was obtained the mixture of 5,10-dihydro-5-[2-(8-vinyloxycarbonyl-8-azabicyclo[3.2.1]oct-3-ylidene)-acetyl]-11H-dibenzo[b,e] [1,4]-diazepin-11-one and 5,10-dihydro-5-[2-(3,4-dehydro-8-vinyloxycarbonyl-8-azabicyclo[3.2.1]oct-3-yl)-acetyl]-11H-dibenzo[b,e] [1,4]-diazepin-11-one.
TLC : (ethylacetate - cyclohexane 60:40) RF = 0.28 and 0.30 on Silicagel plates.

c) A suspension of the mixture of 5,10-dihydro-5-[2-(1-benzyloxycarbonyl-4-piperidinylidene)-acetyl]-11H-dibenzo[b,e] [1,4]-diazepin-11-one and 5,10-dihydro-5-[2-(1-benzloxycarbonyl-1,2,5,6-tetrahydro-4-pyridinyl)-acetyl]-11H-dibenzo[b,e] [1,4]-diazepin-11-one (5.4 g) in methanol (80 ml) was hydrogenated at room temperature and atmospheric pressure in the presence of 10% Pd/C (0.55 g). After the theoretical absorption the catalyst was filtered off, the reaction mixture was concentrated to dryness and the residual material triturated with diethyl ether. 3.34 g. M.p. 254°-257°C.
Analogously was prepared:
5,11-dihydro-11[2-(4-piperidinyl)-acetyl]-6H-pyrido[2,3-b] [1,4]-benzodiazepin-6-one. M.p. 222°-225°C.

Similarly, when the hydrogenation reaction was carried out in the presence of $PtO_2$ and hydrochloric acid, was prepared:
5,10-dihydro-5-[2-(3-piperidinyl)acetyl]-11H-dibenzo[b,e] [1,4]-diazepin-11-one. M.p. 96°-97°C.

Example 10

4,9-dihydro-4-[2-(4-piperidinyl)acetyl]-10H-thieno[3,4-b] [1,5]-benzodiazepin-10-one and
4,9-dihydro-4-[2-(4-piperidinylidene)acetyl]10H-thieno[3,4-b] [1,5]-benzodiazepin-10-one and
4,9-dihydro-4-[2-(1,2,5,6-tetrahydro-4-pyridinyl)acetyl]-10H-thieno [3,4-b] [1,5]-benzodiazepin-10-one

A solution of the mixture of isomers of 4,9-dihydro-4-[2-(1-benzyloxycarbonyl-4-piperidinylidene)acetyl]-10H-thieno[3,4-b [1,5]-benzodiazepin-10-one and 4,9-dihydro-4-[2-(1-benzyloxycarbonyl-1,2,5,6-tetrahydro-4-pyridinyl)-acetyl]-10H-thieno[3,4-b] [1,5]-benzodiazepin-10-one (8.0 g) in formic acid (100 ml) was cautiously added on 10% Pd/C (3.0 g) under nitrogen atmosphere. The resulting suspension was warmed to 70°C for 24 hrs under stirring, then the catalyst was filtered off and the clear solution was concentrated to dryness. The dark brown oil was purified by flash chromatography technique (eluent methylene chloride - methanol - 32% $NH_4OH$ 80:20:2) over Silicagel. 4,9-dihydro-4-[2-(4-piperidinylidene)acetyl]-10H-thieno[3,4-b] [1,5]-benzodiazepin-10-one was eluted first. 0.4 g. M.p. 205°-208°C.
4,9-dihydro-4-[2-(1,2,5,6-tetrahydro-pyridinyl)acetyl]-10H-thieno [3,4-b] [1,5]-benzodiazepin-10-one was eluted second. 0.45 g. M.p. M.p. 198°-201°C.
4,9-dihydro-4-[2-(4-piperidinyl)acetyl]-10H-thieno[3,4-b] [1,5]-benzodiazepin-10-one was eluted third. 0.85 g. M.p. 210°-212°C.

Example 11

5,10-dihydro-5-[2-(8-azabicyclo[3.2.1]oct-3-yl)acetyl]-11H-dibenzo [b,e] [1,4]-diazepin-11-one

a) To a solution of a mixture of isomers of 5,10-dihydro-5-[2-(8-vinyloxycarbonyl-8-azabicyclo[3.2.1] oct-3-ylidene)acetyl]-11H-dibenzo[b,e] [1,4]-diazepin-11-one and 5,10-dihydro-5-[2-(3,4-dehydro-8-vinyloxycarbonyl-8-azabicyclo[3.2.1]oct-3-yl) acetyl]-11H- dibenzo[b,e] [1,4]-diazepin-11-one (2.2 g) in dry dioxane (80 ml) dry hydrochloric acid was bubbled in until saturation. The reaction mixture was then saturated to dryness and the residue dissolved in ethanol. The resulting solution was kept at 60°C for 1 hour, then again concentrated to dryness. The residue was dissolved in acidic water and the phase was washed with ethylacetate. The aqueous phase was made alkaline by potassium carbonate and extracted with ethyl acetate. Evaporation of the solvent left 1,5 g of a mixture of 5,10-dihydro-5-[2-(8-azabicyclo[3.2.1]oct-3-ylidene)acetyl]-11H-dibenzo[b,e] [1,4]-diazepin-11-one and 5,10-dihydro-5-[2-(3,4-dehydro-8-azabicycio [3.2.1]oct-3-yl)acetyl]-11H-dibenzo[b,e] [1,4]-diazepin-11-one.
TLC: (methylene chloride - methanol - 32% $NH_4OH$ 80:20:2)
Rf = 0.45 nd 0.47.

b) 1.4 g of the mixture of 5,10-dihydro-5-[2-(8-azabicyclo[3.2.1] oct-3-ylidene)acetyl]-11H-dibenzo[b,e] [1,4]-diazepin-11-one and 5,10-dihydro-5-[2-(3,4-dehydro-8-azabicyclo[3.2.1] oct-3-yl) acetyl]-11H-dibenzo[b,e] [1,4]diazepin-11-one was dissolved in methanol (70 ml) and hydrogenated at room temperature and atmosphere pressure in the presence of 10% Pd/C (0,25 g) to give 1,25 g of the title compound. M.p. 231°-233°C.

Example 12

4,9-dihydro-4-[2-(8-azabicyclo-[3.2.1]-oct-3-yl)acetyl]-10H-thieno[3,4-b] [1,5]-benzodiazepin-10-one

a) N-vinyloxycarbonyl tropine (34 g) in methylene chloride (1 lt) was reacted at room temperature with pyridinium trifluoro acetate (13 g) and pyridinium dichromate (67 g). After the exothermic reaction had subsided, stirring was continued for further 3 hrs. The reaction mixture was then filtered over Silicagel, washed with diluted HCl and concentrated to dryness. 13.6 g of 8-vinyl-oxycarbonyl-8-azabicyclo(3.2.1)-octan-3-one were obtained. M. p. 53°-55°C.

b) To a suspension of 80% sodium hydride (2.3 g) in 50 ml of anhydrous tetrahydrofurane diethylethoxycarbonylmethylphosphonate (18.9 g) in THF (100 ml) was added dropwise at room temperature. Stirring was continued for further 15 min after the addition was over, then the reaction mixture was cooled to 0°C before the addition of 8-vinyloxycarbonyl-8-azabicyclo(3.2.1)octan-3-one (15 g) in 50 ml of THF. Stirring was continued for further $1\frac{1}{2}$ hrs at 0°C, then the reaction was quenched with water and concentrated to dryness. The resulting oil was taken up in ethyl acetate, washed with water and again concentrated to dryness. 23.5 g of sufficiently pure 8-vinyloxycarbonyl-8-azabicyclo(3.2.1)oct-3-ylidene-acetic acid, ethyl ester were obtained as brownish oil. IR ($cm^{-1}$) 1720-1705 (broad) 1655-1645 (broad).

c) 8-vinyloxycarbonyl-8-azabicyclo(3.2.1)oct-3-ylidene acetic acid, ethyl ester (12.5 g) was dissolved in dry dioxane and saturated with HCl. The reaction mixture was then concentrated to dryness, taken up with absolute ethanol and warmed to 60°C for 30 min. Ethanol was removed under vacuum, the raw material was taken up with water, made alkaline with 10% NaOH and extracted with diethyl ether. After evaporation of the solvent 6.2 g of sufficiently pure 8-azabicyclo(3.2.1)oct-3-ylideneacetic acid, ethyl ester were obtained IR ($cm^{-1}$) 1720, 1655.

d) 8-azabicyclo(3.2.1)oct-3-ylideneacetic acid, ethyl ester (6.2 g) was dissolved in methanol (150 ml) and hydrogenated at room temperature and atmospheric pressure over $PtO_2$ (0.5 g). 4.7 g of 8-azabicyclo(3.2.1)oct-3-ylacetic acid, ethyl ester were obtained. Thick oil IR ($cm^{-1}$) 1735.

e) 8-azabicyclo(3.2.1)oct-3-ylacetic acid, ethyl ester (4.1 g) was reacted with benzylchloroformate (4.25 g) in the presence of triethylamine (3.15 g) in 110 ml of THF at room temperature. After 2 hrs the reaction mixture was concentrated to dryness, taken up in ethylacetate, washed with diluted HCl, diluted $NaHCO_3$ and again concentrated to dryness. 6.2 g of 8-benzyloxycarbonyl-8-azabicyclo(3.2.1)oct-3-ylacetic acid, ethyl ester as a reddish oil were obtained. IR ($cm^{-1}$) 1735,1705.

f) 8-benzyloxycarbonyl-8-azabicyclo(3.2.1)oct-3-ylacetic acid, ethyl ester (8.5 g) was treated with 85% potassium hydroxide (3.4 g) in ethanol (85 ml). After 30 min a solid separated which was collected by filtration. The solid was dissolved in water, the solution was washed with ethyl acetate, acidified with diluted HCl and extracted with ethyl acetate. After evaporation of the solvent 6 g of 8-benzyloxycarbonyl-8-azabicyclo(3.2.1)oct-3-ylacetic acid were obtained. IR ($cm^{-1}$) 1730, 1705.

g) 8-benzyloxycarbonyl-8-azabicyclo(3.2.1)oct-3-ylacetic acid (4.3 g) was dissolved in $CHCl_3$ (100 ml) and reacted with thionyl chloride (3.37 g) at the reflux temperature. The reaction mixture was concentrated to dryness and crude 8-benzyloxycarbonyl-8-azabicyclo (3.2.1)oct-3-ylacetylchloride was used as such. 3.5 g. IR : 1800, 1700 (broad).

h) 8-benzyloxycarbonyl-8-azabicyclo(3.2.1)oct-3-ylacetylchloride (6.3 g)was dissolved in dry dioxane (65 ml) and to the resulting solution 4,9-dihydro-10H-thieno[3,4-b] [1,5]-benzodiazepin-10-one (2.96 g) was added portionwise under stirring. The temperature was risen to 60°C while triethylamine (3 ml) was slowly added; stirring was continued for a further hour. The reaction mixture was then concentrated to dryness, taken up in ethylacetate and washed with water. After evaporation of the solvent the raw material was purified by flash chromatography technique (eluent ethylacetate - cyclohexane 6:4). 4.7 g of 4,9-dihydro-4-[2-(8-benzyloxycarbonyl-8-azabicyclo[3.2.1]oct-3-yl)acetyl]-10H-thieno[3,4-b] [1,5]-diazepin-10-one were obtained. M.p. 187°-190°C.

i) 3.7 g of 4,9-dihydro-4-[2-(8-benzyloxycarbonyl-8-azabicyclo [3.2.1]oct-3-yl)acetyl]-10H-thino[3,4-b] [1,5]-benzodiazepin-10-one were dissolved in formic acid (40 ml)and added over 2 g of 10% Pd/C under an inert atmosphere. The reaction mixture was warmed to 80°C under stirring and kept for two days. The catalyst was filtered off, formic acid was evaporated and the raw material was taken up in water and washed with ethyl acetate. The aqueous phase was extracted with ethyl acetate after treatment with 10% NaOH and after evaporation of the solvent 2.4 g of pure title compound were obtained. M.p. 212-214°C.

Example 13

5,10-dihydro-5- [(4-piperidinyl)amino carbonyl]-11H-dibenzo[b,e] [1,4]-diazepin-11-one

a) A suspension of 5,10-dihydro-5-chlorocarbonyl-11H-dibenzo[b,e] [1,4]-diazepin-11-one (4.2 g) and 1-benzyl-4-aminopiperidine (5.86 g) in dry dioxane (150 ml) was heated under stirring to 60°C for two hrs. After cooling the reaction mixture was filtered and the solids discarded. The mother liquors were concentrated to dryness and trituration with water afforded 6.5 g of 5,10-dihydro-5-[(1-benzyl-4-piperidinyl)-aminocarbonyl]-11H-dibenzo[b,e] [1,4]-diazepin-11-one after filtration and dessication. M.p. 119°-120°C.

Similarly was prepared:

5,10-dihydro-5-[(carbobenzyloxypiperazin-4-yl)carbonyl]-11H-dibenzo[b,e] [1,4]-diazepin-11-one. M.p. 178°-180°C.

b) A suspension of 5,10-dihydro-5-[(1-benzyl-4-piperidinyl)-amino carbonyl]-11H-dibenzo[b,e] [1,4]-diazepin-11-one (5.5 g) in methanol (120 ml) was hydrogenated at room temperature and atmospheric pressure in the presence of 10% Pd/C (1.1 g) for seven days, after filtration and evaporation of the solvent 3.17 g of the title compound were recovered. M.p. 142°-143°C.

Similarly was prepared:

5,10-dihydro-5[(piperazin-4-yl)carbonyl]-11H-dibenzo[b,e] [1,4]-diazepin-11-one. M.p. 138°-140°C.

Example 14

5,10-dihydro-5-[(3-piperidinyl)amino carbonyl]-11H-dibenzo[b,e] [1,4]-diazepin-11-one

a) 5,10-dihydro-5-chlorocarbonyl-11H-dibenzo[b,e] [1,4]-diazepin-11-one (5.0 g) in dry dioxane (200 ml) was warmed to 70°C under stirring and 3-aminopyridine (2.58 g) dissolved in the same solvent (25 ml) was added dropwise. Stirring was continued for 30 min then the reaction mixture was concentrated to dryness. The residue was taken up into acidic water, washed with ethylacetate and made alkaline by sodium carbonate; the aqueous phase was then extracted with methylene chloride from which, after drying, pure 5,10-dihydro-5-[(3-pyridyl)amino carbonyl]-11H-dibenzo[b,e] [1,4]-diazepin-11-one crystallized out. Yield 1.4 g. The hydrochloride salt was obtained from diethyl ether. M.p. 160°C.

b) 5,10-dihydro-5-[(3-pyridyl)amino carbonyl]-11H-dibenzo[b,e] [1,4]-diazepin-11-one hydrochloride (1.3 g) was dissolved in methanol (50 ml) and water (15 ml) and hydrogenated at room temperature and atmospheric pressure in the presence of $PtO_2$ (0.6 g). After evaporation of the solvent and basic workup 1.1 g of title compound were obtained. M.p. 170°C.

Example 15

5,10-dihydro-5-[(4-piperidinyl)carbonyl]-11H-dibenzo[b,e] [1,4]-diazepin-11-one

a) A mixture of 5,0-dihydro-11H-dibenzo[b,e] [1,4]diazepin-11-one (21.0 g), isonicotinoylchloride hydrochloride (23.0 g) and pyridine (21 ml) in dry dioxane (300 ml) was heated to reflux for 6 hrs, then left overnight. The reaction mixture was worked up as usual to afford 40 g of raw material which was crystallized from methanol (19.2 g). The hydrochloride salt of 5,10-dihydro-5-[(4-pyridyl)carbonyl]-11H-dibenzo[b,e] [1,4]-diazepin-11-one was crystallized from ethanol. M.p. 236°-238°C.

Similarly was prepared:

5,10-dihydro-5-[(3-pyridyl)carbonyl]-11H-dibenzo[b,e] [1,4]-diazepin-11-one M.p. 240°C, hydrochloride.

b) 5,10-dihydro-5-[(4-pyridyl)carbonyl]-11H-dibenzo[b,e] [1,4]- diazepin-11-one hydrochloride(10.9 g)was suspended in a mixture of water (100 ml) and methanol (150 ml) and hydrogenated at room temperature and atmospheric pressure in the presence of platinum dioxide (0.5 g). After filtration and evaporation of the solvents 10,5 g of the title compound as hydrochloride salt were obtained. After basification of the aqueous solution the pure base crystallized. M.p. 291°-292°C. M.p. 280°-281°C.

Similarly was prepared:

5,10-dihydro-5-[(3-piperidinyl)carbonyl]-11H-dibenzo[b,e] [1,4]-diazepin-11-one

Example 16

5,6-dihydro-11-[(1-piperazinyl)carbonylmethyl]-11H-dibenz[b,e]-azepin-6-one

a) A suspension of cis-trans 5,6-dihydro-11H-dibenz[b,e]-azepin-6-oxo-11-methylencarboxylic acid (3 g) was suspended in thionyl chloride (20 ml) and stirred at room temperature for 3 hrs. Evaporation of the solvent left the crude acid chloride which was dissolved in dry tertahydrofurane (30 ml) and added to a mixture of carbobenzyloxypiperazine (2,33 g) and triethylamine (1.59 g) dissolved in the same solvent (45 ml). The resulting suspension was stirred at room temperature overnight then the reaction mixture was evaporated to dryness. The residue was taken up into ethylacetate and washed with diluted hydrochloric acid, sodium bicarbonate solution and water. After evaporation of the solvent 3.55 g of 5,6-dihydro-cis-trans-11-[(4-carbobenzyloxypiperazin-1-yl)carbonylmethylen]-11H-dibenz[b,e]-azepin-6-one were obtained. M.p. 106°-108°C.

b) A solution of 5,6-dihydro-cis-trans-11-[(4-carbobenzyloxypiperazin-1-yl)carbonylmethylen]-11H-dibenz[b,e]-azepin-6-one (2.45 g) in methanol (35 ml) was hydrogenated at room temperature and atmospheric pressure in the presence of $PtO_2$ (0.3 g). After ten days,filtration and evaporation of the solvent left a raw material which was purified by flash chromatography (eluent: methylene chloride, methanol, 32% $NH_4OH$ 90:10:1). 0.7 g. M.p. 111°-112°C.

Example 17

5,6-dihydro-cis-trans-11-[(piperidin-4-yl)oxycarbonylmethylen]-11H-dibenz[b,e]-azepin-6-one

a) 80% Sodium hydride (0.04 g) was added portionwise to a solution of N-carbobenzyloxy-4-hydroxy-piperidine (1.59 g) in dry DMF (3.5 ml); after the hydrogen evolution had stopped this alcoholate solution was added to a solution of cis-trans-5,6-dihydro-11H-dibenz[b,e]-azepin-6-oxo-11-methylencarboxylic acid (2.0 g) activated with carbonyldiimidazole (1.22 g), in dry DMF (12 ml). The reaction mixture was stirred at room temperature for $2\frac{1}{2}$ hrs then it was poured into water a solid separated, which was recovered by filtration and washed with water. After chromatography (eluent: toluene/ethyl acetate 7:3) 1.32 g of 5,6-dihydro-cis-trans-11-[(1-carbobenzyloxypiperidin-4-yl)oxycarbonylmethylen]-11H-dibenz[b,e]-azepin-6-one. M.p. 82°-83° C.

Starting from the appropriate acid derivative, was similarly prepared:

5,6-dihydro-11-[(1-carbobenzyloxypiperidin -4-yl)oxycarbonylmethyl]-11H- dibenz[b,e]-azepin-6-one. M.p. 74°-75° C.

b) To a solution of 5,6-dihydro-cis-trans-11-[(1-carbobenzyloxypiperidin-4-yl)oxycarbonylmethylen]-11H-dibenz[b,e]-azepin-6-one (2.32 g) in dry acetonitrile (40 ml), sodium iodide (1.44 g) was added. Trimethylchlorosilane (1.83 g) was subsequently added dropwise while stirring and the resulting mixture was stirred for 18 hrs at room temperature. The reaction mixture was then concentrated to dryness and the pure title compound (1.36 g) was obtained by flash chromatography (eluent methylene chloride-methanol- 32% $NH_4OH$ 90:10:1). M.p. 132°-133° C. Similarly was prepared

5,6-dihydro-11-[(piperidin-4-yl)oxycarbonylmethyl]-11H-dibenz [b,e]-azepin-6-one. M.p. 127°-128° C.

Example 18

5,10-dihydro-5-(4-aminobutyryl)-11H-dibenzo[b,e] [1,4]-diazepin-11-one

a) A suspension of 5,10-dihydro-5-(4-chlorobutyryl)-11H-dibenzo [b,e] [1,4]-diazepin-11-one (20.0 g) and potassium phthalimide (12.9 g) in dry DMF (120 ml) was stirred for 3 hrs at 100° C. The cooled reaction mixture was then poured into water where a semi-solid material separated. Extraction with ethylacetate and evaporation of the solvent left 16.3 g of 5,10-dihydro-5-[4-(N-phthaloyl)butyryl]-11H-dibenzo[b,e] [1,4]diazepin-11-one sufficiently pure. M.p. 255°-258° C.

In a similar way were also prepared:

5,10-dihydro-5-[3-(N-phthaloyl)propionyl]-11H-dibenzo[b,e] [1,4] diazepin-11-one. M.p. 250°-252° C.

5,10-dihydro-5-[2-(N-phthaloyl)acetyl]-11H-dibenzo[b,e] [1,4]-diazepin-11-one. M.p. 245°-247° C.

b) 85% hydrazine hydrate (3.0 g) was added to a suspension of 5,10-dihydro-5-[4-(N-phthaloyl)-butyryl]-11H-dibenzo[b,e] [1,4]-diazepin-11-one (16,3 g) in methanol (400 ml) and the whole was stirred to a gentle reflux for 3 hrs. Methanolic hydrochloric acid was then added to the cooled reaction mixture which was further stirred for 30 min at room temperature. After filtration the solids were discarded, the mother liquors were concentrated to dryness and the residue was purified by flash chromatography (eluent: methylene chloride - methanol -32% $NH_4OH$ 70:30:2). Yield: 9.5 g of the title compound. M.p. 125°-127° C.

Similarly were prepared:

5,10-dihydro-5-(3-aminopropionyl)-11H-dibenzo[b,e] [1,4]-diazepin-11-one. M.p. 195°-198° C.

5,10-dihydro-5-(2-aminoacetyl)-11H-dibenzo[b,e] [1,4] -diazepin-11-one. M.p. 192°-194° C.

Example 19

Xanthene-9-carboxylic acid, 2-amino-ethyl ester

a) 2-(N-benzyloxycarbonyl)aminoethanol (2.34 g) and triethylamine (1.33 g) were dissolved in dry tetrahydrofurane and the resulting solution was stirred at room temperature while xanthene-9-carbonylchloride (3.0 g) was dropped in. Stirring was continued for 20 hrs, then the reaction mixture was concentrated to dryness and worked up as usual. 4.1 g of xanthene-9-carboxylic acid, 2-(N-benzyloxycarbonyl)amino-ethyl ester were afforded. M.p. 130°-132° C.

b) A solution of xanthene-9-carboxylic acid, 2-(N-benzyloxycarbonyl) amino-ethyl ester (3.2 g) in ethanol (35 ml) was hydrogenated in the presence of 10% Pd/C (0.5 g) and of the stoichiometetric amount of hydrochloric acid. Filtration and evaporation of the solvent left the pure title compound as the hydrochloride salt. M.p. 168°-170° C.

Example 20

9-[(3-piperidinyl)methyl]-thioxanthene

9-[(N-methylpiperidin-3-yl)methyl]-thioxanthene (1.93 g) was dissolved in chloroform (10 ml) and anhydrous potassium carbonate (4.3 g) was added. While stirring at room temperature ethylchloroformate (10.1 g) was dropped in at room temperature; as the addition was over the temperature was raised to 60°C and stirring was continued overnight. The cooled reaction mixture was then filtered, the solids discarded and mother liquors concentrated to dryness. Partitioning of the residue between diluted hydrochloric acid and diethyl ether afforded 1.3 g of the intermediate 9-[(N-ethoxycarbonyl)methyl]-thioxanthene which was reacted in 48% hydrobromic acid to afford 0.88 g of the title compound. Thick oil.

Example 21

5,6-dihydro-11-[(4-piperidinyl-methyl)-oxy carbonyl]-11H-dibenz [b,e]-azepin-6-one.

a) Borane-tetrahydrofuran complex (1 M solution in THF, 26 ml) was added slowly to a cold solution (-15°C) of 1-CBZ-4-piperidinyl carboxylic acid (7 g) in THF (15 ml) and stirred at room temperature for one night. The reaction mixture was quenched cautiously by addition of water. Then potassium carbonate (5.5 g) was added and the solvent was removed in vacuo. The residue was taken up with ethyl acetate and washed with aqueous NaCl solution. The organic layer was dried and evaporated in vacuo to give 1-CBZ-4-piperidinyl-methanol (5.5 g) as a thick oil.

b) N,N′-carbonyliimidazole (1.6 g) was added, under nitrogen, to a solution of 5,6-dihydro-6-oxo-dibenz[b,e]-azepin-11-carboxylic acid (2.5 g) in dry dimethylformamide (20 ml), and the mixture was heated at 40°C for 30 min. Then, a mixture of 1-CBZ-4-piperidinyl-methanol (3.7 g) and 1,8-diazabicyclo[5.4.0]undec-7-ene (2.25 g) in dry DMF (20 ml) was added. The reaction mixture was heated for one night at 40°C. After cooling, the solvent was removed in vacuo. The residue was poured into water and solid precipitates which was collected by filtration giving the pure 5,6-dihydro-11-[(1-CBZ-4 piperidinyl-methyl)-oxy carbonyl]-11H-dibenz[b,e]-azepin-6-one (4.5 g). M.p. 185°C (dec.).

c) A solution of 5,6-dihydro-11-[(1-CBZ-4-piperidinyl)-methyl)oxy carbonyl]-11H-dibenz[b,e]-azepin-6-one (4.2 g) in methanol (60 ml) was hydrogenated at room temperature and atmospheric pressure in the presence of 10% Pd/C. The catalyst was then filtered off and the solvent removed in vacuo. The title compound was obtained after trituration with diisopropyl ether (3 g). M.p. 222°C(dec.).

Example 22

5,6-dihydro-11-[(4-piperidinyl)-amino -carbonyl]-11H-dibenz[b,e]-azepin-6-one.

a) Sodium cyanoborohydride (11.4 g) was added to a solution of 1-CBZ-4-piperidone (6 g) and ammonium acetate (19.8 g) in methanol (90 ml). The mixture was stirred at room temperature for 30 min. The reaction mixture was quenched with 2 M HCl and the solvent was removed in vacuo. The residue was partitioned between NaOH and methylene chloride. The combined extracts were washed with water, dried and evaporated in vacuo, giving the pure 1-CBZ-4-amino-piperidine (4.8 g) as an oil.

b) N,N′-carbonyldiimidazole (2 g) was added, under nitrogen, to a solution of 5,6-dihydro-6-oxo-dibenz[b,e]-azepin-11-carboxylic acid (3.12 g) in dry THF (90 ml), and the mixture was heated at 40°C for 30 min. Then, a solution of 1-CBZ-4-amino-piperidine (4.5 g) in THF (50 ml) was added. The reaction mixture was heated for one night at 40°C. After cooling the solvent was removed in vacuo. The residue was poured into water and a solid precipitates which was collected by filtration giving the pure 5.6-dihydro-11-[(1-CBZ-4-piperidinyl)-amino -carbonyl]-11H-dibenz[b,e]-azepin-6-one (4.8 g). M.p. 192°C (dec.).

c) A solution of 5,6-dihydro-11-[(1-CBZ-4-piperidinyl)-amino -carbonyl]-11H-dibenz[b,e]-azepin-6-one (4 g) in ethanol (120 ml) was hydrogenated at room temperature and atmospheric pressure in the presence of 10% Pd/C. The catalyst was then filtered off and the solvent removed in vacuo. The title compound was obtained after trituration with diisopropyl ether (2.5 g) M.p. 219°C (dec).

Analoguosly the following compound was prepared:

5,6-dihydro-11-(piperazinyl-carbonyl)-11H-diabenz[b,e]-azepin-6-one. M.p. 205°C (dec.).

Example 23

5,11-dihydro-11-[2-(4-N-nitroguanyl-piperazin-1-yl)-acetyl]-6H-pyrido[2,3-b] [1,4]-benzodiazepin-6-one

A solution 5,11-dihydro-11-[2-(piperazin-1-yl)-acetyl]-6H-pyrido [2,3-b] [1,4]-benzodiazepin-6-one (7.8 g) and 2-methyl-1(3)-nitro-2-thiopseudourea (3.1 g) in methanol (70 ml) was stirred overnight at room temperature. The solid which separated was collected by filtration and recrystallized from ethanol to give 4.7 g of the title compound. M.p. 200°C (dec.).

By following the same procedure and by utilizing the suitable intermediates the following compounds were also obtained:

5,10-dihydro-11-[2-(4-N-nitroguanyl-piperazin-1-yl)-acetyl]-4-methyl-11H-dibenzo[b,e] [1,4]-diazepin-11-one. M.p. 214°-217°C.

5,10-dihydro-11-[2-(4-N-nitroganyl-piperazin-1-yl)-acetyl]-6-chloro-11H-dibenzo[b,e] [1,4]-diazepin-11-one. M.p. 206°-208°C.

5,10-dihydro-5-[2-(4-N-nitroguanyl-piperazin-1-yl)-acetyl]-11H-dibenzo[b,e] [1,4]-diazepin-11-one. M.p. 210°C.

5-[2-(4-N-nitroguanyl-piperazin-1-yl)-acetyl]-5H-dibenz[b,f]-azepine. M.p. 218°-221°C.

10,11-dihydro-5-[2-(4-N-nitroguanyl-piperazin-1-yl)-acetyl]-5H-dibenz[b,f]-azepine. M.p. 224°-225°C.

5,11-dihydro-3-chloro-11-[2-(4-N-nitroguanyl-piperazin-1-yl)-acetyl]-6H-pyrido[2,3-b] [1,4]-benzodiazepin-6-one. M.p. 196°-200°C (dec.).

4,9-dihydro-4-[2-(4-N-nitroguanyl-piperazin-1-yl-)-acetyl]-10H-thieno[3,4-b] [1,5]-benzodiazepin-10-one. M.p. 213°-216°C.

5,6-dihydro-11-[2-(4-N-nitroguanyl-piperazin-1-yl)-ethyliden]-11H-dibenz[b,e]-azepine-6-one. M.p. 189°-193°C (dec.).

5,6-dihydro-5-[2-(4-N-nitroguanyl-homopiperazin-1-yl)-acetyl]-11H-dibenzo[b,e] [1,4]-diazepin-11-one. M.p. 207°-210°C.

5,6-dihydro-11-[(4-N-nitroguanyl-piperazin-1-yl)-carbonylmethylen]-11H-dibenz[b,e]-azepin-6-one. M.p. 180°-181° C.

5,6-dihydro-11-[(4-N-nitroguanyl-piperazin-1-yl)-carbonylmethyl]-11H-dibenz[b,e]-azepin-6-one. M.p. 248°-249°C.

5,6-dihydro-11-[(1-N-nitroguanyl-piperidin-4-yl)-oxycarbonylmethyl]-11H-dibenz[b,e]-azepin-6-one. M.p. 150°-151°C.

5,10-dihydro-5-[(1-N-nitroguanyl-piperidin-3-yl)-carbonyl]-11H-dibenzo[b,e] [1,4]-diazepin-11-one. M.p. > 250°C.

5,10-dihydro-5-[(1-N-nitroguanyl-piperidin-3-yl)-aminocarbonyl]-11H-dibenzo[b,e] [1,4]-diazepin-11-one. M.p. 270°C.

5,10-dihydro-5-[(1-N-nitroguanyl-piperidin-4-yl)-aminocarbonyl]-11H-dibenzo[b,e] [1,4]-diazepin-11-one. M.p. 253°-255°C.

5,10-dihydro-5-[(1-N-nitroguanyl-piperidin-4-yl)-carbonyl]-11H-dibenzo[b,e] [1,4]-diazepin-11-one. M.p. 222°-223°C.

4,9-dihydro-4-[2-(8-N-nitroguanyl-8-azabicyclo[3.2.1]-octan-3-yl)-acetyl]-10H-thieno[3,4-b] [1,5]-benzodiazepin-10-one. M.p. 198°-200°C.

5,10-dihydro-5-[2-(1-N-nitroguanyl-piperidin-4-yl)-acetyl]-11H-dibenzo[b,e] [1,4]-diazepin-11-one. M.p. 175°C-178°C.

4,9-dihydro-4-[2-(1-N-nitroguanyl-1,2,5,6-tetrahydropyridyn-4-yl)-acetyl]-10H-thieno[3,4-b] [1,5]-benzodiazepin-10-one. M.p. 148°-151°C.

4,9-dihydro-4-[2-(1-N-nitroguanyl-piperidin-4-yl)-acetyl]-10H-thieno[3,4-b] [1,5]-benzodiazepin-10-one. M.p. 180°C (dec.).

4,9-dihydro-4-[2-(1-N-nitroguanyl-4-piperidiniylden)-acetyl]-10H-thieno[3,4-b] [1,5]-benzodiazepin-10-one. M.p. 190°-193°C.

5,11-dihydro-11-[2-(1-N-nitroguanyl-piperidin-4-yl)-acetyl]-6H-pyrido-[2,3-b] [1,4]-benzodiazepin-6-one.

M.p. 204°-206°C.

5,6-dihydro-11-[(1-N-nitroguanyl-piperidin-4-yl)-oxycarbonylmethylen]-11H-dibenz[b,e]-azepin-6-one. M.p. 238°-239°C.

5,10-dihydro-5-[2-(1-N-nitroguanyl-piperidin-3-yl)-acetyl]-11H-dibenzo[b,e] [1,4]-diazepin-11-one. M.p. 98°C (dec.).

5,10-dihydro-5-[3-(N-nitroguanyl-amino)-propionyl]-11H-dibenzo [b,e] [1,4]-diazepin-11-one. M.p. 273°C.

5,10-dihydro-5-[2-(N-nitroguanyl-amino)-acetyl]-11H-dibenzo[b,e] [1,4]-diazepin-11-one. M.p. 230°C.

5,10-dihydro-5-[(1-N-nitroguanyl-piperazin-4-yl)-carbonyl]-11H-dibenzo[b,e] [1,4]-diazepin-11-one. M.p. 210°-212°C.

5,10-dihydro-5-[2-(8-N-nitroguanyl-8-azabicyclo[3.2.1]-octan-3-yl)-acetyl]-11H-dibenzo[b,e] [1,4]diazepin-11-one. M.p. 250°-252°C.

Example 24

5,10-dihydro-5-[(1-N-nitroguanyl-piperidin-3-yl)-oxycarbonyl]-11H-dibenzo[b,e] [1,4]-diazepin-11-one.

To a solution of 1-(N-nitroguanyl)-3-hydroxy piperidine (1.0 g) (obtained from N-nitro-S-methythiopseudourea and 3-hydroxy piperidine) in dry DMF (25 ml), the stoichiometric amount of 80% sodium hydride (0.16 g) was added portionwise and the suspension was stirred until the hydrogen generation ceased. To this solution was added 5,10-dihydro-5-chlorocarbonyl-11H-dibenzo[b,e] [1,4]-diazepin-11-one (1.9 g) and the whole was stirred overnight at room temperature. The reaction mixture was then poured into water and a solid separated which was recovered by filtration. Pure title compound was obtained by flash chromatography technique (eluent: methylene chloride-methanol 90:10) Yield 0.21 g. M.p. 215°C (dec.).

Example 25

5,6-dihydro-11-[(1-N-nitroguanyl-4-piperidinyl)-oxycarbonyl]-11H-dibenz[b,e]-azepin-6-one

a) A solution of 4-hydroxy-piperidine (10 g) and 2-methyl-1(3)nitro-2-thiopseudourea (11.1)g in ethanol (100 ml) and water (50 ml) was heated at 45°C for 5 hrs. The reaction mixture was cooled at room temperature and evaporated to dryness. An ion-exchange resin Amberhyst 15 (Janssen) was added to a solution of the crude residue in methanol and after filtration of the resin and concentration of the solvent in vacuo, the pure 1-nitro-guanyl-4-hydroxy-piperidine was obtained (14.8 g) M.p. 126°C (dec.).

Analoguosly 1-nitro- guanyl -3-hydroxy-piperidine was prepared. M.p. 101°C (dec.).

b) N,N′-carbonyldiimidazole (9 g) was added, under nitrogen, to a solution of 5,6-dihydro-6-oxo-dibenz[b,e]-azepin-11-carboxylic acid (5.75 g) in dry dimethylformamide (75 ml), and the mixture was heated at 40°C for 30 min. Then a mixture of 1-nitro- guanyl-4-hydroxy-piperidine (9.9 g) and 1,8-diazabicyclo[5.4.0] undec-7-ene (7.6 g) in dry dimethylformamide (75 ml) was added. The reaction mixture was heated for another hour at 40°C. After cooling, the solvent was removed in vacuo, and the residue was purified by flash chromatography (eluent: methylene chloride -methanol 95:5) to give the title compound. 10.5 g. M.p. 230° C (dec.).

The following compounds were prepared according to the methods of Example 24:

5,6-dihydro-11-[(1-N-nitroguanyl-3-piperidinyl)oxycarbonyl]-11H-dibenz[b,e]-azepin-6-one. M.p. 233°C (dec.).

5,6-dihydro-11-[(2-nitro-imino-1,3-diazacyclohexane)-oxycarbonyl]-11H-dibenz[b,e]-azepin-6-one. M.p. 245°C (dec.).

Example 26

5,10-dihydro-5-[2-(4-guanyl-piperazin-1-yl)-acetyl]-4-methyl-11H-dibenzo[b,e] [1,4]-diazepin-11-one

(Compound 1)

A solution of 5,10-dihydro-4-methyl-5-[2-(4-N-nitroguanyl-piperazin-1-yl)-acetyl]-11H-dibenzo[b,e] [1,4]-diazepin-11-one (1 g) in formic acid (15 ml) was dropped cautiously on 10% Pd/C (0.5 g). The reaction mixture was heated at 40°C for 5 hrs, filtered, and evaporated to dryness. The crude residue was dissolved in ethanol, then gaseous hydrochloric acid was introduced. From the cooled solution, the dihydrochloride of the title compound crystallized out as a white solid. 0.75 g. M.p.>240°C.

M S (C.I.): 387 m/e $[M + H]^+$

Analysis $C_{21}H_{26}Cl_2N_6O_2$
Found %
C 54.01 H 5.68 N 17.98
Calc. %
C 54.20 H 5.63 N 18.06

According to the above described procedure the following compounds have been obtained:

5,10-dihydro-6-chloro-5-[2-(4-guanyl-piperazin-1-yl)-acetyl]-11H-dibenzo[b,e] [1,4]-diazepin-11-one

(Compound 2)
Dihydrochloride salt (ethanol). M.p.>240°C.
M S (C.I.): 413 m/e $[M + H]^+$
Analysis $C_{20}H_{23}Cl_3N_6O_2$
Found %
C 49.91 H 4.75 N 17.45
Calc. %
C 49.45 H 4.77 N 17.30

5,10-dihydro-5-[2-(4-guanyl-piperazin-1-yl)-acetyl]-11H-dibenzo [b,e] [1,4]-diazepin-11-one

(Compound 3)
Dihydrochloride salt (ethanol). M.p. 243°-245°C (dec.).
M S (C.I.): 379 m/e $[M + H]^+$
Analysis $C_{20}H_{24}Cl_2N_6O_2$
Found %
C 52.98 H 5.46 N 18.35
Calc. %
C 53.21 H 5.36 N18.62

5,11-dihydro-11-[2-(4-guanyl-piperazin-1-yl)-acetyl]-6H-pyrido[2,3-b] [1,4]-benzodiazepin-6-one

(Compound 4)
Dihydrochloride salt (ethanol. M.p. 229°-230°C (dec.).
M S (C.I.): 380 m/e $[M + H]^+$
Analysis $C_{19}H_{23}Cl_2N_7O_2$
Found %
C 50.11 H 5.22 N 21.50
Calc. %
C. 50.44 H 5.12 N 21.68

5-[2-(4-guanyl-piperazin-1-yl)-acetyl]-5H-dibenz[b,f]-azepine

(Compound 5
Diformate salt (diethylether - acetone). M.p. 82°-84°C (dec.).
M S (C.I.): 362 m/e $[M + H]^+$
Analysis $C_{23}H_{27}N_5O_5$
Found %
C 60.54 H 6.22 N 15.07
Calc. %
C 60.91 H 6.00 N 15.44

5,10-dihydro-5-[2-(4-guanyl-piperazin-1-yl)-acetyl]-5H-dibenz[b,f]-azepine

(Compound 6)
Diformate salt (diethylether - acetone). M.p. 65°-70°C (dec.).
M S (C.I.): 364 m/e $[M + H]^+$
Analysis $C_{23}H_{29}N_5O_5$
Found %
C 60.28 H 6.59 N 15.04
Calc. %
C 60.64 H 6.42 N 15.38

5,11-dihydro-3-chloro-11-[2-(4-guanyl-piperazin-1-yl)-acetyl]-6H-pyrido[2,3-b] [1,4]-benzodiazepin-6-one

(Compound 7)
Dihydrochloride salt (ethanol). M.p. 235°-238°C (dec.).
M S (C.I.): 414 m/e [M + H]+
Analysis $C_{19}H_{22}Cl_3H_7O_2$
Found %
C 47.04 H 4.60 N 19.89
Calc. %
C 46.88 H 4.55 N 20.14

4,9-dihydro-4-[2-(4-guanyl-piperazin-1-yl)-acetyl]-10H-thieno [3,4-b] [1,5]-benzodiazepin-10-one

(Compound 8)
Diformate salt (diethyl ether). M.p. 84°-90°C (dec.).
M S (C.I.): 385 m/e [M + H]+
Analysis $C_{20}H_{24}N_6O_6S$
Found %
C 50.33 H 5.13 N 17.41
Calc. %
C 50.41 H 5.08 N 17.63

5,6-dihydro-11-[2-(4-guanyl-piperazin-1-yl)ethyliden]-11H-dibenz [b,e]-azepine-6-one

(Compound 9)
Dihydrochloride salt (isopropanol). M.p. >245°C.
M S (C.I.): 362 m/e [M + H]+
Analysis $C_{21}H_{25}Cl_2N_5O$
Found %
C 57.86 H 5.71 N 16.01
Calc. %
C 58.07 H 5.80 N 16.12

5,10-dihydro-5-[2-(4-guanyl-homopiperazin-1-yl)-acetyl]-11H-dibenzo[b,e] [1,4]-diazepin-11-one

(Compound 10)
Dihydrochloride salt (ethanol). M.p. 205°-207°C (dec.).
M S (C.I.): 392 m/e [M + H]+
Analysis $C_{21}H_{25}Cl_2N_6O_2$
Found %
C 58.70 H 5.59 N 19.71
Calc. %
C 58.94 H 5.65 N 19.64

5,6-dihydro-11-[(4-guanyl-piperazin-1-yl)-carbonylmethylen]-11H-dibenz[b,e]-azepine-6-one

(Compound 11)
Formate salt (diethyl ether). M.p. 120°-121°C (dec.).
M S (C.I.): 376 m/e [M + H]+
Analysis $C_{22}H_{23}N_5O_4$
Found %
C 62.36 H 5.62 N 16.50
Calc. %
C 62.69 H 5.50 N 16.62

5,6-dihydro-11-[(4-guanyl-piperazin-1-yl)-carbonylmethyl]-11H-dibenz[b,e]-azepine-6-one

(Compound 12)
Formate salt (diethyl ether). M.p. 89°-91°C (dec.).
M S (C.I.) : 378 m/e [M + H]+
Analysis $C_{22}H_{25}N_5O_4$
Found %
C 62.12 H 6.04 N 16.44

Calc. %
C 62.40 H 5.95 N 16.54

5,6-dihydro-11-[(1-guanyl-piperidin-4-yl)-oxycarbonyl-methyl]-11H-dibenz[b,e]-azepine-6-one

(Compound 13)
Formate salt (ethanol). M.p. 246°-247° C (dec.).
M S (C.I.) : 393 m/e $[M + H]^+$
Analysis $C_{23}H_{26}N_4O_5$
Found %
C 62.85 H 6.06 N 12.59
Calc. %
C 63.00 H 5.98 N 12.78

5,10-dihydro-5-[(1-guanyl-piperidin-3-yl)-carbonyl]-11H-dibenzo [b,e] [1,4]-diazepin-11-one.

(Compound 14)
Formate salt (ethanol - diethyl ether). M.p. 196°-199°C (dec.).
M S (C.I.): 364 m/e $[M + H]^+$
Analysis $C_{21}H_{23}N_5O_4$
Found %
C 61.42 H 5.69 N 17.00
Calc. %
C 61.60 H 5.66 N 17.11

5,10-dihydro-5-[(1-guanyl-piperidin-4-yl)-aminocarbonyl]-11H-dibenzo[b,e] [1,4]-diazepin-11-one.

(Compound 15)
Formate salt (diethyl ether). M.p. 207°-210°C (dec.).
M S (C.I.) : 379 m/e $[M + H]^+$
Analysis $C_{21}H_{24}N_6O_4$
Found %
C 59.18 H 5.78 N 19.54
Calc. %
C 59.42 H 5.69 N 19.80

5,10-dihydro-5-[(1-guanyl-piperidin-3-yl)-aminocarbonyl]-11H-dibenzo[b,e] [1,4]-diazepin-11-one.

(Compound 16)
Formate salt (ethanol - diethyl ether ). M.p. 190°C (dec.).
M S (C.I.) : 379 m/e $[M + H]^+$
Analysis $C_{21}H_{24}N_6O_4$
Found %
C 59.31 H 5.80 N 19.58
Calc. %
C 59.42 H 5.69 N 19.80

5,10-dihydro-5-[(1-guanyl-piperidin-4-yl)-carbonyl]-11H-dibenzo [b,e] [1,4]-diazepin-11-one.

(Compound 17)
Hydrochloride salt (ethanol). M.p. 235°C (dec.).
M S (C.I.) : 364 m/e $[M + H]^+$
Analysis $C_{20}H_{22}ClN_5O_2$
Found %
C 60.07 H 5.55 N 17.52
Calc. %
C 60.18 H 5.53 N 17.52

5,10-dihydro-5-[(1-guanyl-piperidin-3-yl)-oxycarbonyl]-11H-dibenzo[b,e] [1,4]-diazepin-11-one

(Compound 18)
Formate salt (acetone - diethyl ether). M.p. 110°-140°C (dec.)
M S (C.I.) : 380 m/e $[M + H]^+$
Analysis $C_{21}H_{23}N_5O_5$
Found %

C 59.08 H 5.60 N 16.15
Calc. %
C 59.28 H 5.45 N 16.46

4,9-dihydro-4-[2-(8-guanyl-8-azabicyclo[3.2.1]-octan-3-yl)-acetyl]-10H-thieno[3,4-b] [1,5]-benzodiazepin-10-one

(Compound 19)
Formate salt (diethyl ether). M.p. 60°-64°C (dec.).
M S (C.I.) : 410 m/e $[M + H]^+$
Analysis $C_{22}H_{25}N_5O_4S$
Found %
C 57.78 H 5.63 N 15.02
Calc. %
C 58.00 H 5.53 N 15.37

5,10-dihydro-5-[2-(1-guanyl-piperidin-4-yl)-acetyl]-11H-dibenzo b,e [1,4]-diazepin-11-one.

(Compound 20)
Hydrochloride salt (acetone -diethyl ether). M.p. 115°-118°C (dec.)
M S (C.I.) : 378 m/e $[M + H]^+$
Analysis $C_{21}H_{24}ClN_5O_2$
Found %
C 60.77 H 5.81 N 17.03
Calc. %
C 60.93 H 5.84 N 16.92

4,9-dihydro-4-[2-(1-guanyl-1,2,5,6-tetrahydropyridin-4-yl)-acetyl]-4H-thieno[3,4-b] [1,5]-benzodiazepin-10-one

(Compound 21)
Hydrochloride salt (ethanol - diethyl ether). M.p. 173°-178°C (dec.)
M S (C.I.) : 382 m/e $[M + H]^+$
Analysis $C_{19}H_{20}ClN_5O_2S$
Found %
C 54.29 H 4.75 N 16.80
Calc. %
C 54.60 H 4.82 N 16.76

4,9-dihydro-4-[2-(1-guanyl-piperidin-4-yl)-acetyl]-4H-thieno [3,4-b] [1,5]-benzodiazepin-10-one

(Compound 22)
Hydrochloride salt (water). M.p. 205°-208°C (dec.).
M S (C.I.) : 384 m/e $[M + H]^+$
Analysis $C_{19}H_{22}ClN_5O_2S$
Found %
C 54.15 H 5.33 N 16.50
Calc. %
C 54.34 H 5.28 N 16.68

4,9-dihydro-4-[2-(1-guanyl-4-piperidinyliden)-acetyl]-4H-thieno [3,4-b] [1,5]-benzodiazepin -10-one

(Compound 23)
Hydrochloride salt (ethanol - diethyl ether). M.p. 198°-204°C (dec)
M S (C.I.) : 382 m/e $[M + H]^+$
Analysis $C_{19}H_{20}ClN_5O_2S$
Found %
C 54.25 H 4.89 N 16.54
Calc. %
C 54.60 H 4.82 N 16.76

5,11-dihydro-11-[2-(1-guanyl-piperidin-4-yl)-acetyl]-6H-pyrido [2,3-b] [1,4]-benzodiazepin-6-one.

(Compound 24)
Hydrochloride salt. (ethanol) M.p. 183°-186°C.
M S (C.I.) : 379 m/e [M + H]$^+$
Analysis $C_{20}H_{23}ClN_6O_2$
Found %
C 57.60 H 5. 62 N 20.31
Calc. %
C 57.89 H 5. 59 N 20.26

5,6-dihydro-11-[(1-guanyl-piperidin-4-yl)-oxycarbonyl-methylen]-11H-dibenz[b,e]-azepin-6-one.

(Compound 25)
Hydrochloride salt (ethanol - diethyl ether). M.p. 200°-204°C (dec.) M S (C.I.) : 391 m/e [M + H]$^+$
Analysis $C_{22}H_{23}ClN_4O_3$
Found %
C 66.51 H 5.88 N 14.03
Calc. %
C 66.64 H 5.84 N 14.13

5,10-dihydro-5-[2-(1-guanyl-piperidin-3-yl)-acetyl]-11H-dibenzo [b,e] [1,4]-diazepin-11-one.

(Compound 26)
Hydrochloride salt (acetone - diethyl ether). M.p. 145°-148°C.
M S (C.I.) : 378 m/e [M + H]$^+$
Analysis $C_{21}H_{24}ClN_5O_2$
Found %
C 60.65 H 5.94 N 16.69
Calc. %
C 60.93 H 5.84 N 16.92

5,10-dihydro-5-[3-(N-guanyl-amino)-propionyl]-11H-dibenzo[b,e] [1,4]-diazepin-11-one.

(Compound 27)
Hydrochloride salt (acetone). M.p. 238°-243°C (dec.).
M S (C.I.) : 324 m/e [M + H]$^+$
Analysis $C_{17}H_{18}ClN_5O_2$
Found %
C 56.67 H 5.12 N 19.31
Calc. %
C 56.74 H 5.04 N 19.47

5,10-dihydro-5-[2-(N-guanyl-amino)-acetyl]-11H-dibenzo[b,e] [1,4]-diazepin-11-one.

(Compound 28)
Hydrochloride salt (ethanol). M.p. 230°-232°C.
M S (C.I.) : 310 m/e [M + H]$^+$
Analysis $C_{16}H_{16}ClN_5O_2$
Found %
C 55.32 H 4.75 N 20.09
Calc. %
C 55.57 H 4.66 N 20.25

5,10-dihydro-5-[(1-guanyl-piperazin-4-yl)-carbonyl]-11H-dibenzo [b.e] [1,4]-diazepin-11-one

(Compound 29)
Hydrochloride salt (acetone - diethyl ether). M.p. 206°-209°C (dec.)
M S (C.I.) : 365 m/e [M + H]$^+$
Analisys $C_{19}H_{21}ClN_6O_2$
Found %
C 56.79 H 5.35 N 20.88
Calc. %
C 56.92 H 5.98 N 20.97

5,10-dihydro-5-[2-(8-guanyl-8-azabicyclo[3.2.1]-octan-3-yl)-acetyl]-11H-dibenzo[b,e] [1,4]-diazepin-11-one.

(Compound 30)
Formate salt (diethyl ether). M.p. 160°C (dec.).
M S (C.I.) : 404 m/e M + H $^+$
Analysis $C_{24}H_{27}N_5O_4$
Found %
C 64.01 H 6.13 N 15.42
Calc. %
C 64.12 H 6.05 N 15.58

5,6-dihydro-11-[(1-guanyl-4-piperidinyl)-oxycarbonyl]-11H-dibenz[b,e]-azepin-6-one.

(Compound 31)
Hydrochloride salt (acetonitrile). M.p. 215°C.
M S (C.I.) : 379 m/e $[M + H]^+$
Analisys $C_{21}H_{23}ClN_4O_3$
Found %
C 60.77 H 5.63 N 13.46
Calc. %
C 60.79 H 5.59 N 8.55

5,6-dihydro-[(1-guanyl-3-piperidinyl)-oxycarbonyl]-11H-dibenz[b,e]-azepin-6-one

(Compound 32)
Hydrochloride salt (acetonitrile). M.p. 167°C.
M S (C.I.) : 379 m/e $[M + H]^+$
Analisys $C_{21}H_{23}ClN_4O_3$
Found %
C 60.75 H 5.56 N 13.50
Calc. %
C 60.79 H 5.59 N 13.50

5,6-dihydro-11-[(2-imino-1,3-diazacyclohexan-5-yl)-oxycarbonil]-11H-dibenz[b,e]-azepin-6-one.

(Compound 33)
Hydrochloride salt (acetonitrile). M.p. 320°C.
M S (C.I.) : 351 m/e $[M + H]^+$
Analysis $C_{19}H_{19}ClN_4O_3$
Found %
C 60.03 H 4.90 N 14.55
Calc. %
C 58.99 H 4.95 N 14.48

Example 27

4,9-dihydro-4-[2-(4-guanyl-homopiperazin-1-yl)-acetyl]-10H-thieno[3,4-b] [1,5] -benzodiazepin-10-one.

(Compound 34)
A mixture of 4,9-dihydro-4-[2-(homopiperazin-1-yl)-acetyl]-10H-thieno[3,4-b] [1,5]-benzodiazepin-10-one dihydrochloride (0.72 g) and cyanamide (0.14 g) in water (0.15 ml) was heated at 100°C for 3 hrs. The residue was dissolved in water, and picric acid was added; the picrate salt was filtered, suspended between concentrated hydrochloric (20 ml) and benzene (60 ml). The suspension was heated at 80°C until two clear layers were obtained. The aqueos layer was separated and evaporated to dryness. The crude hydrochloride of the title compound so obtained was purified by crystallization from ethanol. 0.25 g. M.p. 228°-230°C (dec.).
M S (C.I.) : 399 m/e $[M + H]^+$
Analysis $C_{19}H_{24}Cl_2N_6O_2S$
Found %
C 48.08 H 5.19 N 17.60
Calc. %
C 48.40 H 5.13 N 17.83

The following compounds were also prepared according to the above described method:

5,10-dihydro-5-[2-(4-guanyl-2,5-dimethyl-piperazin-1-yl)-acetyl]-11H-dibenzo[b,e] [1,4]-dazepin-11-one.

(Compound 35)
Dihydrochloride salt (ethanol - diethyl ether). M.p. 240°-242°C (dec.)
M S (C.I.) : 407 m/e $[M + H]^+$
Analysis $C_{22}H_{28}Cl_2N_6O_2$
Found %
C 54.88 H 5.95 N 17.40
Calc. %
C 55.11 H 5.88 N 17.53

4,9-dihydro-4-[2-(4-guanyl-2,5-dimethyl-piperazin-1-yl)-acetyl]-10H-thieno[3,4-b] [1,5]-benzodiazepin-10-one

(Compound 36)
Dihydrochloride salt (ethanol - diethyl ether). M.p. 215°-216°C (dec.)
M S (C.I.): 413 m/e $[M + H]^+$
Analysis $C_{20}H_{26}Cl_2N_6O_2S$
Found %
C 49.66 H 5.46 N 17.04
Calc. %
C 49.48 H 5.40 N 17.31

5,11-dihydro-11-[2-(4-guanyl-2,5-dimethyl-piperazin-1-yl)acetyl]-6H-pyrido[2,3-b] [1,4]-benzodiazepin-6-one.

(Compound 37)
Dihydrochloride salt (ethanol - diethyl ether) M.p. 172°-178°C (dec.)
M S (C.I.): 408 m/e $[M + H]^+$
Analysis $C_{21}H_{27}Cl_2N_7O_2$
Found %
C 52.41 H 5.71 N 20.18
Calc. %
C 52.50 H 5.66 N 20.41

5,11-dihydro-11-[2-(4-guanyl-homopiperazin-1-yl)-acetyl]-6H-pyrido[2,3b] [1,4]-benzodiazepin-6-one

(Compound 38)
Dihydrochloride salt (ethanol - diethyl ether). M.p. 201°-207°C (dec.)
M S (C.I.): 394 m/e $M + H^+$
Analysis $C_{20}H_{25}Cl_2N_7O_2$
Found %
C 51.40 H 5.43 N 20.85
Calc. %
C 51.50 H 5.40 N 21.03

9-[N-guanyl-(2-amino ethoxy-carbonyl)]-xanthene

(Compound 39)
Hydrochloride salt (ethanol -diethyl ether). M.p. 80°-85°C (dec.)
M S (C.I.): 312 m/e $[M + H]^+$
Analysis $C_{17}H_{18}ClN_3O_2$
Found %
C 58.53 H 5.24 N 11.97
Calc. %
C 58.69 H 5.21 N 12.08

9-[(1-guanyl-piperidin-3-yl)-methyl]-thioxanthene

(Compound 40)
Hydrochloride salt (acetone). M.p. 190°-193°C.
M S (C.I.) : 338 m/e $[M + H]^+$
Analysis $C_{20}H_{24}ClN_3S$
Found %
C 64.09 H 6.58 N 11.05
Calc. %

C 64.24 H 6.74 N 11.24

5,6-dihydro-11-[(1-guanyl-4-piperidinyl-methyl)-oxycarbonyl]-11H-dibenz[b,e]-azepin-6-one

(Compound 41)
Formate salt (acetonitrile). M.p. 241°C.
M S (C.I.) : 393 m/e $[M + H]^+$
Analysis $C_{23}H_{26}N_4O_5$
Found %
C 63.08 H 5.99 N 12.71
Calc. %
C 63.00 H 5.98 N 12.78

5,6-dihydro-11-[(1-guanyl-4-piperidinyl)-amino -carbonyl]-11H-dibenz[b,e]-azepin-6-one.

(Compound 42)
Formate salt (acetonitrile). M.p.252°C
M S (C.I.): 378 m/e $[M + H]^+$
Analysis $C_{22}H_{25}N_5O_4$
Found %
C 62.51 H 6.04 N 16.47
Calc. %
C 62.40 H 5.95 N 16.54

5,6-dihydro-11-[(1-guanyl-4-piperazinyl)-carbonyl]-11H-dibenz[b,e]-azepin-6-one.

(Compound 43)
Formate salt (acetonitrile) M.p. 204°C
M S (C.I.) : 364m/e $[M + H]^+$
Analysis $C_{21}H_{23}N_5O_4$
Found %
C 61.50 H 5.58 N 16.97
Calc. %
C 61.60 H 5.66 N 17.10

Example 28

5,10-dihydro-5-[2-(4-guanyl-piperazin-1-yl)-acetyl]-11H-dibenzo[b,e] [1,4]-diazepin-11-one.

(Compound 3)
A solution of 5,10-dihydro-5-[2-(piperazin-1-yl)-acetyl]-11H-dibenzo[b,e][1,4]-diazepin-11-one (1.2 g) and 5-methyl isothiourea sulphate (0.8 g) in ethanol (30 ml) was refluxed for 24 hrs. The solution was evaporated to dryness and after flash-column chromatography (eluent : metanol - acetic acid - ammonium hydroxide 100:2:3) the crude title compound was obtained. It was purified through its picrate salt from which the hydrochloride was easily obtained. 0.55 g. M.p. 241°-243°C (dec.) (from ethanol).
M S (C.I.) : 379 m/e $[M + H]^+$
Analysis $C_{20}H_{24}Cl_2N_6O_2$
Found %
C 53.02 H 5.31 N 18.70
Calc. %
C 53.21 H 5.36 N 18.62
In an analogous manner the following compounds were obtained:

5,11-dihydro-11-[2-(4-guanyl-piperazin-1-yl)-acetyl]-6H-pyrido[2,3-b] [1,4]-benzodiazepin-6-one.

(Compound 4)
Dihydrochloride salt (ethanol). M.p. 220°-230°C (dec.)
M S (C.I.) : 380 m/e $[M + H]^+$
Analysis $C_{19}H_{23}Cl_2N_7O_2$
Found %
C 50.21 H 5.23 N 21.57
Calc. %
C 50.44 H 5.12 N 21.68

5,10-dihydro-5-[4-(N-guanyl-amino)-butyryl]-11H-dibenz[b,e] [1,4]-diazepin-11-one.

(Compound 44)
Hydrochloride salt (ethanol -diethyl ether). M.p. 162°-168°C (dec.)
M S (C.I.) : 338 m/e $[M + H]^+$
Analysis $C_{18}H_{20}ClN_5O_2$
Found %
C 57.60 H 5.43 N 18.61
Calc. %
C 57.83 H 5.39 N 18.74

Example 29

5,11-dihydro-3-chloro-11-[2-(4-guanyl-piperazin-1-yl)-acetyl]-6H-pyrido[2,3-b] [1,4]-benzodiazepin-6-one.

(Compound 7)
A solution of 5,11-dihydro-3-chloro-11-[2-(4-N-nitroguanyl-piperazin-1-yl)-acetyl]-6H-pyrido-[2,3-b][1,4]-benzodiazepin-6-one (1.5 g) in formic acid (30 ml) was heated at 50°C for 8 hrs in the presence of $SnCl_2{\bullet}2H_2O$ (5.9 g); the reaction mixture was filtered and then evaporated to dryness. Water (30 ml) and 37% HCl (3 ml) were added and $H_2S$ was bubbled into this solution; the SnS which separated was filtered, and from the solution evaporated to dryness the crude title compound was obtained. This was purified by crystallization from absolute ethanol. 0.35 g. M.p. 235°-238°C (dec.)
M S (C.I.) : 414 m/e $[M + H]^+$
Analysis $C_{19}H_{22}Cl_3N_7O_2$
Found %
C 46.73 H 4.62 N 20.01
Calc. %
C 46.88 H 4.55 N 20.14

Example 30

5,10-dihydro-5-[2-(4-N-methyl-thiocarbamyl-piperazin-1-yl)-acetyl]-11H-dibenzo[b,e] [1,4]-diazepin-11-one.

A solution of 5,10-dihydro-5-[2-(piperazin-1-yl)-acetyl]-11H-dibenzo[b,e] [1,4]-diazepin-11-one (0.5 g) and methylisothiocyanate (0.2 g) in methanol was refluxed for 1 hour. The solution was cooled at room temperature and evaporated to dryness. The pure title compound was obtained after crystallization of the residue from absolute ethanol. 0.4 g. M.p. 235°-236°C.

By following the above described procedure, and by utilyzing the suitable intermediates, these compounds were obtained:

5,11-dihydro-11-[2-(4-N-methylthiocarbonyl-piperazin-1-yl)-acetyl]-6H-pyrido[2,3-b] [1,4]-benzodiazepin-6-one. M.p. 250°-251°C.

5,10-dihydro-5-[2-( 4-N-isopropylthiocarbamyl-piperazin-1-yl)-acetyl]-11H-dibenzo[b,e] [1,4]-diazepin-11-one. M.p. 139°-143°C.

4,9-dihydro-4-[2-(4-N-isopropylthiocarbamyl-piperazin-1-yl)-acetyl]-10H-thieno[3,4-b] [1,5]-benzodiazepin -10-one. M.p. 201°-204°C.

5,10-dihydro-5-[2-(1-N-isopropyl-thiocarbamyl-piperidin-4-yl)-acetyl]-11H- dibenzo[b,e] [1,4]-diazepin-11-one. M.p. 146°-148°C.

5,10-dihydro-5-[2-(1-N-octyl-thiocarbamyl-piperidin-4-yl)-acetyl]-11H-dibenzo[b,e] [1,4]-diazepin-11-one. M.p. 180°-183°C.

5,10-dihydro-5-[2-(4-N-octyl-thiocarbamyl-piperazin-1-yl)-acetyl]-11H-dibenzo[b,e] [1,4]-diazepin-11-one. M.p. 118°-120°C.

4,9-dihydro-4-[2-(4-N-octyl-thiocarbamyl-piperazin-1-yl)-acetyl]-10H-thieno[3,4-b] [1,5]-benzodiazepin-10-one. M.p. 109°-112°C.

5,10-dihydro-5-[(4-N-octyl-thiocarbamyl-piperazin-1-yl)-carbonyl]-11H-dibenzo[b,e] [1,4]-diazepin-11-one. M.p. 150°-152°C.

5,10-dihydro-5-[2-(4-N-t-butyl-thiocarbamyl-piperazin-1-yl)-acetyl]-11H-dibenzo[b,e] [1,4]-diazepin-11-one.

M.p. 135°-138°C.

9-[2-(N-methyl-thioureido)-ethoxycarbonyl]-thioxantene. M.p. 140°-142°C.

Example 31

5,10-dihydro-5-[2-(4-N-methyl-iminomethyl-piperazin-1yl)-acetyl]-11H-dibenzo[b,e] [1,4]-diazepin-11-one.

(Compound 45)

A suspension of 5,10-dihydro-5-[2-(4-N-methyl-thiocarbamyl-piperazin-1-yl)-acetyl]-11H-dibenzo[b,e] [1,4]-diazepin-11-one (0.89 g) and Raney-Nickel (4 g) in dichloromethane was stirred at room temperature for 1 hour; the suspension was filtered, hydrochloric acid in ethanol was added and the clear solution was evaporated to dryness. The crude hydrochloride was purified by crystallization from acetone. 0.24 g. M.p. 182°C (dec.).

M S (C.I.): 378 m/e {M + H]+

Analysis $C_{21}H_{25}Cl_2N_5O_2$

Found %

C 55.80 H 5.63 N 15.41

Calc. %

C 56.00 H 5.59 N 15.55

The following compounds were analogously prepared:

5,10-dihydro-5-[2-(1-N-isopropyl-imino-methyl-piperidin-4-yl)-acetyl]-11H-dibenzo[b,e] [1,4]-diazepin-11-one.

(Compound 46)

Hydrochloride salt (ethanol - ethylacetate). M.p. 204°-206°C (dec)

M S (C.I.) : 405 m/e [M + H]+

Analysis

Found %

C 65.15 H 6.80 N 12.57

$C_{24}H_{29}ClN_4O_2$

Calc. %

C 65.22 H 6.84 N 12.67

4,9-dihydro-4-[2-(4-N-octyl-imino-methyl-piperazin-1-yl)-acetyl]-10H-thieno[3,4-b] [1,4]-benzodiazepin-6-one

(Compound 47)

Dihydrochloride salt (ethanol - ethyl acetate). M.p. 160°-162°C(dec)

M S (C.I.) : 482 m/e[M + H]+

Analysis $C_{26}H_{37}Cl_2N_5O_2S$

Found %

C 56.26 H 6.77 N 12.54

Calc. %

C 56.31 H 6.73 N 12.63

5,10-dihydro-5-[2-(1-N-octyl-imino-methyl-piperidin-4-yl)-acetyl]-11H-dibenzo[b,e] [1,4]-diazepin-11-one.

(Compound 48)

Hydrochloride salt (ethanol - diethyl ether). M.p. 230°-235°C (dec)

M S (C.I.) : 475 m/e [M + H]+

Analysis $C_{29}H_{39}ClN_4O_2$

Found %

C 68.05 H 7.73 N 10.82

Calc. %

C 68.16 H 7.69 N 10.96

5,11-dihydro-11-[2-(4-N-methyl-imino-methyl-piperazin -1-yl)-acetyl]-6H-pyrido-[2,3-b] [1,4]-benzodiazepin-6-one.

(Compound 49)

Dihydrochloride salt (acetone). M.p. 218-220°C (dec.)

M S (C.I.) : 379 m/e [M + H]+

Analysis $C_{20}H_{24}Cl_2N_6O_2$

Found %

C 53.07 H 5.41 N 18.54

Calc. %
C 53.21 H 5.36 N 18.62

Xanthen-9-carboxylic acid-2-[N-methyl-(imino methyl)-amino]-ethyl ester.

(Compound 50)
Hydrochloride salt (diethyl ether). M.p. 70°-78°C (dec)(after liophilization
M S (C.I.) : 311 m/e [M + H]⁺
Analysis $C_{18}H_{19}ClN_2O_3$
Found %
C 62.03 H 5.64 N 7.94
Calc. %
C 62.33 H 5.55 N 8.08

5,10-dihydro-5-[2-(4-N-isopropyl imino methyl-piperazin-1-yl)-acetyl]-11H-dibenzo[b,e] [1,4]-diazepin-11-one.

(Compound 51)
Dihydrochloride salt (ethanol - diethyl ether) M.p. 200°C (dec.)
M S (C.I.): 406 m/e [M + H]⁺
Analysis $C_{23}H_{29}Cl_2N_5O_2$
Found %
C 57.70 H 6.13 N 14.68
Calc. %
C 57.74 H 6.11 N 14.64

5,10-dihydro-5-[2-(4-N-t-butyl-imino-methyl-piperazin-1-yl)-acetyl]-11H-dibenzo[b,e] [1,4]-diazepin 11-one.

(Compound 52)
Dihydrochloride salt (ethanol -diethyl ether). M.p. 230°C (dec.)
M S (C.I.) : 420 m/e [M + H]⁺
Analysis $C_{24}H_{31}Cl_2N_5O_2$
Found %
C 58.47 H 6.40 N 14.08
Calc. %
C 58.53 H 6.34 N 14.22

5,10-dihydro-5-[(4-N-octyl-imino-methyl-piperazin-1-yl)-carbonyl]-11H-dibenzo[b,e] [1,4]-diazepin-11-one.

(Compound 53)
Hydrochloride salt (ethanol - diethyl ether) M.p. 140°-143°C (dec)
M S (C.I.) : 462 m/e [M + H]⁺
Analysis $C_{27}H_{36}ClN_5O_2$
Found %
C 65.06 H 7.31 H 13.98
Calc. %
C 65.11 H 7.29 H 14.06

4,9-dihydro-4[2-(4-N-isopropyl-iminomethyl-piperazin-1-yl)-acetyl]-10H-thieno[3,4-b] [1,5]-benzodiazepin-10-one.

(Compound 54)
Dihydrochloride salt (acetone). M.p. 224°-226°C (dec.)
M S (C.I.) : 412 m/e [M + H]⁺
Analysis $C_{21}H_{27}Cl_2N_5O_2S$
Found %
C 52.00 H 5.65 N 14.39
Calc. %
C 52.06 H 5.62 N 14.46

Example 32

5,6-dihydro-11-[2-(1-iminomethyl-piperazin-4-yl)-ethyliden]-11H-dibenz[b,e]-azepin-6-one .

(Compound 55)

A solution of ethylformimidate hydrochloride (1.8 g) and 5,6-dihydro-11-[2-(1-piperazinyl)-ethyliden]-11H-dibenz[b,e]-azepin-6-one (5 g) in absolute ethanol (50 ml) was stirred overnight at room temperature. The reaction mixture was evaporated to dryness and from the crude residue the pure title compound was obtained after crystallization from isopropanol. 2.2 g. Dihydrochloride salt. M.p. 245°C (dec.)

M S (C.I.): 355 m/e $[M + H]^+$

Analysis $C_{21}H_{24}CL_2N_4O$

Found %

C 59.81 H 6.06 H 13.25

Calc. %

C 60.00 H 5.99 H 13.33

Accordingly the following compound was obtained:

9-[(1-iminomethyl-piperidin-3-yl)-methyl]-thioxanthene

(Compound 56)

Hydrochloride salt (acetone - diethyl ether).M.p. 207°-210°C (dec)

M S (C.I.) : 323 m/e $[M + H]^+$

Analysis $C_{20}H_{23}ClN_2S$

Found %

C 66.85 H 6.51 N 7.77

Calc. %

C 66.93 H 6.46 N 7.80

Example 33

5,10-dihydro-5-[2-(4- α-iminoethyl - piperazin-1-yl)-acetyl]-11H-dibenzo[b,e] [1,4]-diazepin-11-one.

(Compound 57)

A solution of 5,10-dihydro-5-[2-(piperazin-1-yl)-acetyl]-11H-dibenzo[b,e] [1,4]-diazepin-11-one (0.5 g) and ethylacetimidate hydrochloride (0.2 g) in ethanol (15 ml)was stirred overnight at room temperature. The solid, which separated, was collected by filtration and recrystallized from isopropanol to give 0.23 g of the title compound as hydrochloride salt. M.p. 215°C (dec.)

M S (C.I.) : 378 m/e $[M + H]^+$

Analysis $C_{21}H_{25}Cl_2N_5O_2$

Found %

C 55.73 H 5.62 N 15.45

Calc. %

C 56.00 H 5.59 N 15.55

Following the above described procedure, these analogous compounds were also prepared:

5,11-dihydro-11-[2-(4-α-iminoethyl-piperazin-1-yl)-acetyl]-6H-pyrido[2,3-b] [1,4]-benzodiazepin-6-one.

(Compound 58)

Dihydrochloride salt (ethanol - acetone). M.p. 200°-202°C (dec.)

M S (C.I.) : 379 m/e $[M + H]^+$

Analysis $C_{20}H_{24}Cl_2N_6O_2$

Found %

C 53.04 H 5.41 N 18.55

Calc. %

C 53.21 H 5.36 N 18.62

4,9-dihydro-4-[2-(4-α-iminoethyl-piperazin-1 yl)-acetyl]-10H-thieno[3,4-b] [1,5]-benzodiazepin-10-one.

(Compound 59)

Picrate salt (water). M.p.>280°C

M S (C.I.) : 384 m/e $[M + H]^+$

Analysis $C_{31}H_{27}N_{11}O_{16}S$

Found %

C 44.33 H 3.18 N 18.40

Calc. %

C 44.23 H 3.23 N 18.31

5,10-dihydro-5-[2-(1-α-iminoethyl-piperidin-h-yl)-acetyl]-11H-dibenzo[b,e] [1,4]-diazepin-11-one.

(Compound 60)
Hydrochloride salt (acetone). M.p. 125°-130°C (dec.)
M S (C.I.): 377 m/e $[M + H]^+$
Analysis $C_{22}H_{25}ClN_4O_2$
Found %
C 63.66 H 6.15 N 13.40
Calc. %
C 63.99 H 6.10 N 13.57

5,10-dihydro-5-[2-(1-α-iminoethyl - piperidin-3-yl)-acetyl]-11H-dibenzo[b,e] [1,4]-diazepin-11-one.

(Compound 61)
Picrate salt (water). M.p.>280°C.
M S (C.I.) : 377 m/e $[M + H]^+$
Analysis $C_{28}H_{27}N_7O_9$
Found %
C 55.60 H 4.53 N 16.12
Calc. %
C 55.53 H 4.49 N 16.19

5,10-dihydro-5-[(1-α-iminoethyl-piperidin-3-yl)-carbonyl]-11H-dibenzo[b,e] [1,4]-diazepin-11-one.

(Compound 62)
Hydrochloride salt (acetone - diethyl ether) M.p. 130°C (dec.)
M S (Cl.) : 363 m/e $[M + H]^+$
Analysis $C_{21}H_{23}ClN_4O_2$
Found %
C 63.02 H 5.85 N 13.98
Calc. %
C 63.23 H 5.81 N 14.05

9-[(1-α-iminoethyl-piperidin-3-yl)-methyl]-thioxanthene.

(Compound 63)
Hydrochloride salt (ethanol -diethyl ether). M.p. 195°-198°C (dec.)
M S(C.I.) : 337 m/e $[M + H]^+$
Analysis $C_{21}H_{25}ClN_2S$
Found %
C 67.50 H 6.35 N 7.39
Calc. %
C 67.63 H 6.76 N 7.51

Example 34

5,10-dihydro-5-[2-[(4-α-methyl iminoethyl)-piperazin-1-yl]-acetyl]-11H-dibenzo[b,e] [1,4]-diazepin-11-one.

(Compound 64)
A solution of 5,10-dihydro-5-[2- (piperazin-1-yl)-acetyl]-11H-dibenzo[b,e] [1,4]-diazepin-11-one (0.5 g) and N-methyl-phenylacetimidate (0.24 g) in acetone (15 ml) and ethanol (5 ml) was stirred at room temperature overnight. A solution of hydrochloric acid in ethanol was added and the reaction mixture was evaporated to dryness. The pure title compound was obtained after flash chromatography purification (eluent: met OH $-CH_3COOH-NH_4OH$ 100:2:3). 0.4 g. M.p. 224°-227°C (dec.) (acetone).
M S (C.I.) : 392 m/e $[M + H]^+$
Analysis $C_{22}H_{27}Cl_2N_5O_2$
Found %
C 56.82 H 5.90 N 15.01
Calc. %
C 56.89 H 5.86 N 15.08

Example 35

4,9-dihydro-4-[2-(4-N-methylguanyl-piperazin-1-yl)-acetyl]-10H-thieno[3,4-b] [1,5]-benzodiazepin-10-one.

(Compound 65 )

A solution of 4,9-dihydro-4-[2-(piperazin-1-yl)-acetyl]-10H-thieno[3,4-b] [1,5]-benzodiazepin-10-one (1 g) and N,S-dimethylisothiouroniumhydroiodide (0.67 g) in ethanol (10 ml) was stirred overnight at room temperature. The solution was cooled and the separated salt of the title compounds was recovered by filtration. 0.28 g. M.p. 248°-250°C (dec.)

M S (C.I.) : 399 m/e $[M + H]^+$

Analysis $C_{19}H_{23}JN_6O_2S$

Found %

C 43.30 H 4.43 N 15.91

Calc. %

C 43.35 H 4.40 N 15.97

Analogously the following compound was prepared:

5,10-dihydro-5-[2-(4-N-methylguanyl-piperazin-1-yl)-acetyl]-11H-dibenzo[b,e] [1,4]-diazepin-11-one.

(Compound 66)

Dihydrochloride salt (ethanol). M.p. 240°C (dec.)

M S (C.I.) : 393 m/e $[M + H]^+$

Analysis $C_{21}H_{26}Cl_2N_6O_2$

Found %

C 54.04 H 5.66 N 17.98

Calc. %

C 54.19 H 5.63 N 18.06

Example 36

5,10-dihydro-5-[2-(4-N-methyl-N'-isopropyl-guanyl-piperazin-1-yl)-acetyl]-11H-dibenzo[b,e] [1,4]-diazepin-11-one.

(Compound 67)

A solution of 5,10-dihydro-5-[2-(4-N-isopropyl-thicarbamyl-piperazin-1-yl)-acetyl]-11H-dienzo[b,e] [1,4]diazepin-11-one (2 g ) and dimethyl sulphate (0.65 g) in acetonitrile (40 ml) was refluxed for 1 hour, cooled and evaporated to dryness. A 33 % solution of methylamine in ethanol (15 ml) was added to the residue and this reaction mixture was stirred at room temperature for 48 hrs, and evaporated to dryness. The title compound was obtained after purification of the residue by flash-column chromatography technique (eluent : metOH - $CH_3COOH$ -$NH_4OH$ 100:2:3). 0.22 g. Hydrochloride salt (acetone). M.p. 230-234°C (dec.)

M S (C.I.) : 435 m/e $[M + H]^+$

Analysis $C_{24}H_{32}Cl_2N_6O_2$

Found %

C 56.71 H 6.41 N 16.35

Calc. %

C 56.80 H 6.36 N 16.56

Utilizing the same procedure and starting with the suitable intermediates, the following compounds were also obtained:

4,9-dihydro-4-[2-(4-N,N'-dimethylguanyl-piperazin-1-yl)-acetyl]-10H-thieno [3,4-b] [1,5]-benzodiazepin-10-one.

(Compound 68)

Dihydrochloride salt (isopropanol). M.p. 245°-246°C (dec.)

M S (C.I.): 413 m/e $[M + H]^+$

Analysis $C_{20}H_{26}Cl_2N_6O_2S$

Found %

C 49.35 H 5.44 N 17.06

Calc. %

C 49.48 H 5.41 N 17.32

5,10-dihydro-5-[2-(4-N,N'-dimethylguanyl-piperazin-1-yl)-acetyl]-11H-dibenzo[b,e] [1,4]-diazepin-11-one

(Compound 69)
Hydrochloride salt (ethanol - diethyl ether). M.p. 228°-234°C(dec.)
M S (C.I.) : 407 m/e [M + H]+
Analysis $C_{22}H_{28}Cl_2N_6O_2$
Found %
C 54.84 H 5.96 N 17.21
Calc. %
C 55.11 H 5.88 N 17.53

Example 37

5,10-dihydro-5-[2-[4-($\Delta_2$-imidazolin-2-yl )-piperazin-1-yl]-acetyl]-11H-dibenzo[b,e] [1,4]-diazepin-11-one.

(Compound 70)
A solution of 5,10-dihydro-5-[2-(piperazin-1-yl)-acetyl]-11H-dibenzo[b,e] [1,4]-diazepin-11-one (0.5 g) and 2-thiomethyl-4.5-dihydro-imidazole hydroiodide (0.4 g) in ethanol (10 ml) was refluxed for 4 hrs and cooled at room temperature. The crude product, which separated, was collected by filtration and recrystallized from methanol. 0.2 g of the pure title compound were obtained as hydroiodide salt. M.p. 272°C (dec.)
M S (C.I.) : 405 m/e [M + H]+
Analysis $C_{22}H_{25}JN_6O_2$
Found %
C 50.51 H 4.70 N 15.75
Calc. %
C 49.63 H 4.73 N 15.78
The following compounds were accordingly obtained:

9-[[1-($\Delta_2$-imidazolin-2-yl) -piperidin-3-yl-methyl]-thioxanthene

(Compound 71)
Hydroiodide salt (ethanol). M.p. 240°-244°C (dec.).
M S (C.I.) : 365 m/e [M + H]+
Analysis $C_{22}H_{26}JN_3S$
Found %
C 53.54 H 5.30 N 8.63
Calc. %
C 53.77 H 5.33 N 8.55

5,10-dihydro-5-[2-[2-($\Delta_2$-imidazolin-2-yl)-piperidin-4-yl]-acetyl]-11H-dibenzo[b,e] [1,4]-diazepin-11-one.

(Compound 72)
Hydroiodide salt (methanol). M.p. 255°-260°C (dec.)
M S (C.I.) : 404 m/e [M + H]+
Analysis $C_{23}H_{26}JN_5O_2$
Found %
C 52.04 H 5.00 N 13.05
Calc.%
C 51.98 H 4.93 N 13.18

5,11-dihydro-11-[2-1-($\Delta_2$-imidazolin-2-yl )-piperidin-4-yl] -acetyl]-6H-pyrido[2,3-b] [1,4]-benzodiazepin-6-one.

(Compound 73)
Hydrochloride salt (ethanol - acetone). M.p. 138°-140°C
M S (C.I.) : 405 m/e [M + H]+
Analysis $C_{22}H_{25}ClN_6O_2$
Found %
C 59.75 H 5.76 N 18.98
Calc. %
C 51.98 H 5.72 N 19.06

Example 38

5,10-dihydro-5-[2-(4-cyano-piperidin-1-yl)-acetyl]-11H-dibenzo[b,e] [1,4]-diazepin-11-one.
A solution of 5,10-dihydro-5-(2-chloro-acetyl)-11H-dibenzo[b,e] [1,4]-diazepin-11-one (3.44 g), 4-cyano piperidine (2.65 g) and triethylamine (1.2 g) in acetone (40 ml) was stirred at 45°C for 24 hrs. The reaction

mixture was evaporated to dryness and 10% HCl in water and ethyl acetate were added. The aqueous solution was separated, 17% $Na_2CO_3$ solution was added to adjust the pH at 8; the solid which separated was filtered and dried to give the title compound. 3.6 g. M.p. 193°-195°C.

Similarly the following compounds were prepared from the appropriate starting compounds:
4,9-dihydro-4-[2-(4-cyanopiperidin-1-yl)-acetyl]-10H- thieno[3,4-b] [1,5]-benzodiazepin-10-one. M.p. 128°-130°C.

5,10-dihydro-5-[2-(3-cyanopiperidin-1-yl)-acetyl]-11H-dibenzo[b,e] [1,4]-diazepin-11-one. M.p. 90°C.

Example 39

5,10-dihydro-5-[2-(4-guanyl-piperidin-1-yl)-acetyl]-11H-dibenzo[b,e] [1,4]-diazepin-11-one.

(Compound 74)

A mixture of 5,10-dihydro-5-[2-(4-cyano-piperidin-1-yl)-acetyl]-11H-dibenzo[b,e] [1,4]-diazepin-11-one (2 g) and thiourea (2.12 g) was heated at 180°C for 2 hrs. From the crude residue after column chromatography purification (eluent $CH_2Cl_2$-metOH - $NH_4OH$ 90:10:1) the title compound was obtained. 0.28 g.
Dihydrochloride salt. M.p. 100°-105°C (dec.)
M S (C.I.) : 378 m/e $[M + H]^+$
Analysis $C_{21}H_{25}Cl_2N_5O_2$
Found %
C 56.12 H 5.63 N 15.44
Calc. %
C 56.00 H 5.59 N 15.55

The following compounds were also obtained by utilizing the same procedure:

4,9-dihydro-4-[2-(4-guanyl-piperidin-1-yl)-acetyl]-10H-thieno[3,4-b] [1,5]-benzodiazepin-10-one.

(Compound 75)
Dihydrochloride salt (acetone -diethyl ether). M.p. 210°-215°C (dec)
M S (C.I.) : 384 m/e $[M + H]^+$
Analysis $C_{19}H_{23}Cl_2N_5O_2S$
Found %
C 50.13 H 5.14 N 15.21
Calc. %
C 49.99 H 5.08 N 15.35

5,10-dihydro-5-[2-(4-N-methlguanyl-piperidin-1-yl)-acetyl]-11H-dibenzo[b,e] [1,4] -diazepin-11-one.

(Compound 76)
Dihydrochloride salt (acetone). M.p.>270°C.
M S (C.I.) : 392 m/e $[M + H]^+$
Analysis $C_{22}H_{27}Cl_2N_5O_2$
Found %
C 56.70 H 5.94 N 14.98
Calc. %
C 56.89 H 5.86 N 15.08

4,9-dihydro-4-[2-(4-N-methylguanyl-piperidin-1-yl)-acetyl]-10H-thieno[3,4-b] [1,5]-benzodiazepin-10-one.

(Compound 77)
Dihydrochloride salt (isopropanol). M.p. 215° 220°C.
M S (C.I.): 398 m/e$[M + H]^+$
Analysis $C_{20}H_{25}Cl_2N_5O_2S$
Found %
C 60.19 H 5.94 N 17.50
Calc. %
C 60.43 H 5.83 N 17.62

5,10-dihydro-5-[2-(3-N-methylguanyl-piperidin-1-yl)-acetyl]-11H-dibenzo[b,e] [1,4]-diazepin-11-one.

(Compound 78)
Dihydrochloride (acetone). M.p. 240°C (dec).
M S (C.I.) : 392 m/e $[M + H]^+$

Analysis $C_{22}H_{27}Cl_2N_5O_2$
Found %
C 56.95 H 5.94 N 15.01
Calc. %
C 56.89 H 5.86 N 15.08

5,10-dihydro-5-[2-(4-N-t-butyl-guanyl-piperidin-1-yl)-acetyl]-11H-dibenzo[b,e] [1,4]-diazepin-11-one.

(Compound 79)
Dihydrochloride salt (acetone-diethyl ether). M.p. 220°-225°C(dec)
M S (C.I.) : 434 m/e $[M + H]^+$
Analysis $C_{25}H_{33}Cl_2N_5C_3$
Found %
C 59.07 H 6.62 N 13.77
Calc. %
C 59.28 H 6.57 N 13.83

Example 40

5,10-dihydro-5-[2-(4-N-phenyl-guanyl-piperidin-1-yl)-acetyl]-11H-dibenzo[b,e] [1,4]-diazepin-11-one.

(Compound 80)
A mixture of 5,10-dihydro-5-[2-(4-cyano-piperidin-1-yl)-acetyl]-11H-dibenzo[b,e] [1,4]-diazepin-11-one (2 g), aniline (0.5 g) and $AlCl_3$ (0.75 g) in tetrachloro ethane was cautiously heated at 130°-140°C for 1 hour. The reaction mixture was cooled at room temperature, water was added and the two phases were separated. By adding a solution of picric acid in water to the aqueous solution, the crude picrate of the title compound was obtained. This was conventionally converted into its dihydrochloride salt. 0.75 g. M.p. 240°-243°C (dec.).
M S (C.I.) : 454 m/e $[M + H]^+$
Analysis $C_{27}H_{29}Cl_2N_5O_2$
Found %
C 61.44 H 6.01 N 13.16
Calc. %
C 61.59 H 5.55 N 13.31

Example 41

5,10-dihydro-5-[3-(3-amino-propyl-amino)-propionyl]-11H-dibenzo[b,e] [1,4]-diazepin-11-one.

a) A mixture of 5,10-dihydro-5-(3-chloropropionyl)-11H-dibenzo[b,e] [1,4] -diazepin-11-one (6.5 g), N -CBZ-1.3 diamino propane (5 g), and anhydrous $K_2CO_3$ (6.2 g) in THF (200 ml) and DMF (20 ml) was stirred at room temperature for 4 days. The reaction mixture was evaporated to dryness and the intermediate 5,10-dihydro-5-[3-(3N-CBZ-amino-propyl-amino)-propionyl]-11H-dibenzo[b.e] [1,4]-diazepin-11-one was obtained after flash-column chromatography (eluent $CH_2Cl_2$ - MetOH 9:1). 3.5 g. Waxy solid.

b) This product (3 g) was dissolved in absolute ethanol (60 ml) and hydrogenated at room temperature and pressure in the presence of 10% Pd/C (0.5 g) as a catalyzer. As the theoretical amount of hydrogen was taken up the reaction mixture was filtered and evaporated to dryness. The title compound (2.4 g) was obtained as a white thick oil and utilized directly in the following step. It was identified and stored as its hydrochloride salt from ethanol.

By following the same procedure and starting from the suitable intermediates, these compounds were prepared:

5,11-dihydro-11-[3-(3-amino-propyl-amino)-propionyl]-6H-pyrido[2,3-b] [1,4]-benzodiazepin-6-one.
Oxalate salt (ethanol). M.p. 175°-180°C (dec).

4,9-dihydro-4-[3-(3-amino-propyl-amino)-propionyl]-10H-thieno[3,4-b] [1,5]-benzodiazepin-10-one.
Oxalate salt (ethanol). M.p. 188°-191°C (dec.).

Example 42

5,10-dihydro-5-[3-(1,4,5,6-tetrahydropirimidin-1-yl)-propionyl]-11H-dibenzo[b,e] [1,4]-diazepin-11-one.

(Compound 81)

A solution of 5,10-dihydro-5-[3-amino-propyl-amino-propionyl]-11H-dibenzo[b,e] [1,4]-diazepin-11-one (0.8 g) and formamidine acetate (0.25 g) in ethanol (10 ml) was stirred at room temperature for six hrs. The solution was evaporated to dryness, the residue was dissolved in acetone and gaseous hydrochloric acid was introduced.

The hydrochloride of the title compound crystallized out and was filtered and dried. 0.45 g M.p. 120°C (dec.)
M S (C.I.) : 349 m/e $[M + H]^+$
Analysis $C_{20}H_{22}Cl_2N_4O_2$
Found %
C 62.18 H 5.59 N 14.41
Calc. %
C 62.41 H 5.50 N 14.56

Accordingly the following compounds were obtained:

5,11-dihydro-11-[3-(1,4,5,6-tetrahydropirimidin-1-yl)-propionyl]-6H-pyrido[2,3-b] [1,5]-benzodiazepin-6-one.

(Compound 82)
Hydrochloride salt (acetone - diethyl ether) M.p. 114°-116°C (dec.)
M S (C.I.) : 350 m/e $[M + H]^+$
Analysis $C_{19}H_{20}ClN_5O_2$
Found %
C 50.40 H 6.23 N 16.57
Calc. %
C 50.68 H 6.16 N 16.72

4,9-dihydro-4-[3-(1,4,5,6-tetrahydropirimidin-1-yl)-propionyl]-10H-thieno[3,4-b] [1,5]-benzodiazepin-10-one.

(Compound 83)
Hydrochloride salt (acetone - diethyl ether) M.p. 70°-71°C (dec.)
M S (C.I.) : 356 m/e $[M + H]^+$
Analysis $C_{18}H_{19}ClN_4O_2S$
Found %
C 55.08 H 5.01 N 14.20
Calc. %
C 55.30 H 4.90 N 14.33

Example 43

5,10-dihydro-5-[3-(2-methyl-1,4,5,6-tetrahydropirimidin-1-yl)-propionyl]-11H-dibenzo[b,e] [1,4]-diazepin-11-one.

(Compound 84)

A solution of 5,10-dihydro-5-[3-(3-amino-propyl-amino)-propionyl]-11H-dibenzo[b,e] [1,4]-diazepin-11-one (1.8 g) and acetamidine hydrochloride (0.5 g) in ethanol (7 ml) was stirred overnight. The crystallized product was filtered and dried to give 1.4 g of the title compound as hydrochloride salt. M.p. 209°-211°C (dec.). M S (C.I.) : 363 m/e $[M + H]^+$
Analysis $C_{21}H_{23}ClN_4O_2$
Found %
C 63.16 H 5.92 N 13.98
Calc. %
C 63.23 H 5.81 N 14.05

Similarly this product was prepared:

5,11-dihydro-11-[3-(2-methyl-1,4,5,6-tetrahydropirimidin-1-yl)-propionyl]-6H-pyrido[2,3-b] [1,4]-benzodiazepin-6-one.

(Compound 85)
Hydrochloride salt (ethanol). M.p. 210°C (dec.)
M S (C.I.) : 364 m/e $[M + H]^+$
Analysis $C_{20}H_{22}ClN_5O_2$
Found %
C 60.12 H 5.59 N 17.42
Calc. %
C 60.07 H 5.55 N 17.52

Example 44

5,10-dihydro-5-[3-(2-amino-1,4,5,6-tetrahydropirimidin-1-yl)-propionyl]-11H-dibenzo[b,e] [1,4]-diazepin-11-one.

(Compound 86)

a) A solution of 5,10-dihydro-5-[3-(3-amino-propyl-amino)-propionyl]-11H-dibenzo[b,e] [1,4]-diazepin-11-one (2 g) and 2-methyl-1-nitro-2-thiopseudourea (0.8 g) in water was stirred at room temperature for 48 hrs. The solid which separated was filtered and after trituration from diethyl ether 0.95 g of the intermediate 5,10-dihydro-5-[3-(2-nitroamino-1,4,5,6-tetrahydropirimidin-1-yl)-propionyl]-11H-dibenzo[b,e] [1,4]-diazepin-11-one were obtained. M.p. 160°-164°C.

Similarly was prepared:

4,9-dihydro-4-[3-(2-nitroamino-1,4,5,6-tetrahydropirimidin-1-yl)-propionyl]-10H-thieno[3,4-b] [1,5]-benzodiazepin-10-one. M.p. 80°-81°C (dec.)

b) A suspension of 5,10-dihydro-5-[3-(2-nitroamino-1,4,5,6-tetrahydropirimidin-1-yl)-propionyl]-11H-dibenzo[b,e] [1,4]-diazepin- 11-one (0.6 g) and 10% Pd/C (0.6 g) in formic acid (15 ml) was stirred at 45°C for 4 hrs. The reaction mixture was filtered and evaporated to dryness. The formate salt of the desired compound was obtained as a white solid (0.47 g). M.p. 68°-70°C (dec.)
M S (C.I.) : 364 m/e $[M + H]^+$
Analysis $C_{21}H_{23}N_5O_4$
Found %
C 61.80 H 5.59 N 17.24
Calc. %
C 61.60 H 5.66 N 17.11

Accordingly to this procedure the following compound was prepared:

4,9-dihydro-4-[3-(2-amino-1,4,5,6-tetrahydropyrimidin-1-yl)-propionyl]-10H-thieno[3,4-b] [1,5]-benzodiazepin-10-one.

(Compound 87)
Formate salt (diethyl ether). M.p. 58°-62°C (dec.)
M S (C.I.): 370 m/e $[M + H]^+$
Analysis $C_{19}H_{21}N_5O_4S$
Found %
C 54.80 H 5.16 N 16.71
Calc. %
C 54.92 H 5.09 N 16.86

Example 45

9-[(1-methyl-2-amino-1,4,5,6-tetrahydropirimidin-5-yl)-oxycarbonyl]-xanthene

(Compound 88)
Xanthen-9-carbonylchloride (1.5 g) was slowly added to a solution of 1-methyl-2-amino-5-hydroxy-1,4,5,6-tetrahydropirimidine hydrochloride (1 g) in anhydrous DMF (10 ml). The solution was stirred for 2 hrs at room temperature and evaporated to dryness. The title compound was obtained after purification of the crude residue by flash-column chromatography (eluent : $CH_2Cl_2$ - MetOH -$H_2O$ 80:20:2) 0.8 g. M.p. 175°-178°C (dec.)
M S (C.I.) : 338 m/e $[M + H]^+$
Analysis
Found %
C 60.85 H 5.43 N 11.08
$C_{19}H_{20}ClN_3O_3$
Calc. %
C 61.04 H 5.39 N 11.24
Similarly this compound was obtained:

9-[(2-methyl-1,4,5,6-tetrahydropirimidin-5-yl)-oxycarbonyl]-xanthene

(Compound 89)
Hydrochloride salt (acetone - diethyl ether). M.p. 110°-115°C (dec.)
M S (C.I.) : 323 m/e $[M + H]^+$
Analysis $C_{19}H_{19}ClN_2O_3$
Found %
C 63.15 H 5.38 N 7.68
Calc. %
C 63.59 H 5.33 N 7.81

Example 46

9-[(1,2-dimethyl-1,4,5,6-tetrahydropirimidin-5-yl)-oxycarbonyl]-xanthene

(Compound 90)

a) A solution of methyliodide (1.64 g) and 2-methyl-5-hydroxy-1,4,5,6-tetrahydropirimidine (1.2 g) in THF was stirred at room temperature. The solid which evaporated was collected by filtration and crystallized from acetone and diethyl ether to give 0.4 g of 1,2-dimethyl-5-hydroxy-1,4,5,6-tetrahydropirimidin as hydroiodidesalt.

b) Xanthene-9-carbonylchloride (1.43 g) was slowly added to a solution of the above described intermediate (1.5 g) in anhydrous DMF (15 ml). The solution was stirred at room temperature for 2 hrs and evaporated to dryness. The title compound was obtained as hydroiodide salt after purification of the crude residue by flash-column chromatography (eluent $CH_2Cl_2$ - $CH_3OH$ - $H_2O$ 85:15:1.5). 0.35 g. M.p. 248°C (dec.)
M S (C.I.) : 337 m/e $[M + H]^+$
Analysis $C_{20}H_2IN_2O_3$
Found %
C 51.72 H 4.58 N 6.12
Calc. %
C 51.80 H 4.56 N 6.04

Example 47

5,10-dihydro-5-[(2-methyl-1,4,5,6-tetrahydropirimidin-5-yl)-oxycarbonyl]-11H-dibenzo[b,e] [1,4]-diazepin-11-one.

(Compound 91)

a) A solution of 5,10-dihydro-5-chlorocarbonyl-11H-dibenzo[b,e] [1,4]-diazepin-11-one (8.8 g) in anhydrous DMF (70 ml) was slowly dropped into a well stirred suspension of 5-hydroxy-2-methylpirimidine (3.6 g) and 80% NaH (1 g) in anhydrous DMF (50 ml). After 1 hour stirring, water (1500 ml) was added and the reaction mixture was extracted with ethylacetate. From the organic solution the crude 5,10-dihydro-5-[(2-methyl-piperidin-5-yl)-oxycarbonyl]-11H-dibenzo[b,e] [1,4]-diazepin-11-one was obtained. It was purified through its hydrochloride salt in an ethanol - acetone mixture. (6.2 g). M.p. 200°-202°C.

b) This intermediate (5 g) was dissolved in water (25 ml) and ethanol (70 ml) and hydrogenated at room temperature and pressure in the presence of 10% Pd/C (1 g). When the theoretical amount of hydrogen was taken up, the reaction mixture was filtered and evaporated to dryness. The hydrochloride of the title compound was obtained as a white powder after trituration in diethyl ether. 4.1 g. M.p. 183°-186°C.
M S (C.I.) : 351 m/e $[M + H]^+$
Analysis $C_{19}H_{19}ClN_4O_3$
Found %
C 58.71 H 5.04 N 14.59
Calc. %
C 58.99 H 4.95 N 14.48

The following not limitative examples of pharmaceutical compositions according to the invention are reported:

Example 48

Tablets
- active ingredient 20 mg
- lactose 247 mg
- corn starch 30 mg
- magnesium stearate 3 mg

Method of preparation: The active ingredient, lactose, and corn starch were mixed and homogeneously moistened with water. After screening of the moist mass and drying in a tray drier, the mixture was again passed through a screen and magnesium stearate was added. Then the mixture was pressed into tablets weighing 300 mg each. Each tablet contains 20 mg of active ingredient.

Example 49

Freeze-dried vials
- active ingredient 10 mg
- mannitol 50 mg

Method of preparation: the active ingredient and mannitol were dissolved in an appropriate amount of water for injection. The resulting solution was filtered and filled into vials under sterile conditions. The vials were freeze-dried and stopped with a suitable closure.

Example 50

Suppositories
- active ingredient 50 mg
- semisynthetic glicerides of fatty acids 750 mg

Method of preparation: the semisynthetic glicerides of fatty acids were melted and the active ingredient was added while stirring homogeneously. After cooling at a proper temperature the mass was poured into preformed moulds for suppositories weighing 800 mg each. Each suppository contains 50 mg of active ingredient.

Example 51

Oral drops
- active ingredient 10 mg
- sorbitol 350 mg
- propylene glycol 200 mg
- citric acid 1 mg
- sodium citrate 3 mg
- demineralized water q.s. 1 ml

Method of preparation: the active ingredient, citric acid and sodium citrate were dissolved in a mixture of a proper amount of water and propylene glycol. Then sorbitol was added and the final solution was filtered. The solution contains 1% of active ingredient and is administered by using a proper dropper.

Example 52

Capsules
- active ingredient 20 mg
- lactose 178 mg
- Magnesium stearate 2mg

Method of preparation: the active ingredient was mixed with the auxiliary products, and the mixture was passed through a screen and mixed homogeneously in a suitable device. The resulting mixture was filled into hard gelatine capsules (200 mg per capsule); each capsule contains 20 mg of active ingredient.

**Claims**

1. Compounds of general formula (I)

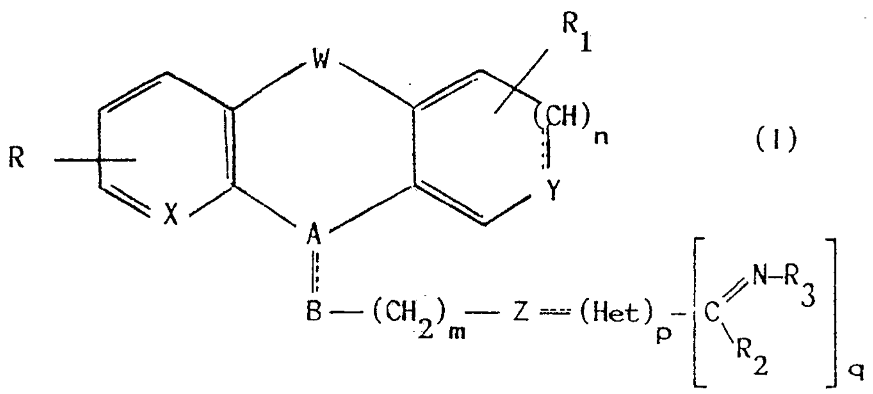

(I)

wherein
R represents a hydrogen atom or a halogen atom
X represents nitrogen or -CH-group
W represents a -NH-CO-, -CH=CH-, $-CH_2-CH_2-$ group, oxygen or sulfur
$R_1$ represents a hydrogen atom or a $C_{1-4}$ alkyl group
n is 0 or 1
Y represents sulfur or a -CH- group
A represents carbon or nitrogen
B represents a -CH- group (provided that A is different from nitrogen), -COO-, -CO-, or $-CH_2-$ group
m is 0 or an integer from 1 to 3
Z represents -NH-, -CO-, -COO- or -CH- group, or it is absent
p is 0 or 1
Het represents piperazinyl, homopiperazinyl, piperidinyl, tropyl or tetrahydropirimidinyl group, each group being optionally substituted by a $C_{1-4}$ alkyl group or an amino group
q is 0 or 1
$R_2$ represents a hydrogen atom, a $C_{1-4}$ alkyl group or an amino group optionally substituted by a linear or branched $C_{1-4}$ alkyl group or phenyl group
$R_3$ represents a linear or branched $C_{1-8}$ alkyl group or a hydrogen atom (provided that the bond between Het and the group

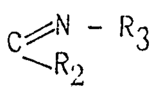

is a carbon -carbon bond or A = C and B = CH)
or
$R_2$and $R_3$ may join together to form a heterocyclic 5-membered ring, tautomers thereof and acid addition salts of the aforesaid compounds.

2 . Physiologically compatible acid addition salts of compounds of formula (I) as claimed in claim 1.

3. Salts according to claim 2 obtained for salification with hydrochloric, hydrobromic, hydroiodic, sulphuric or methansulphonic acid.

4. Pharmaceutical compositions comprising as active ingredient at least one compound of general formula (I), according to claim 1 or a tautomer or physiologically compatible acid addition salt thereof in association with a pharmaceutical carriers or excipients.

5 . Pharmaceutical compositions according to claim 4 for use as antimuscarinic agents.

6. Pharmaceutical compositions according to claim 5 for treatment of patients suffering from motility disorders of the gastrointestinal or urogenital tract and from peptic ulcer disorders.

7. Process for the preparation of compounds of general formula (I)

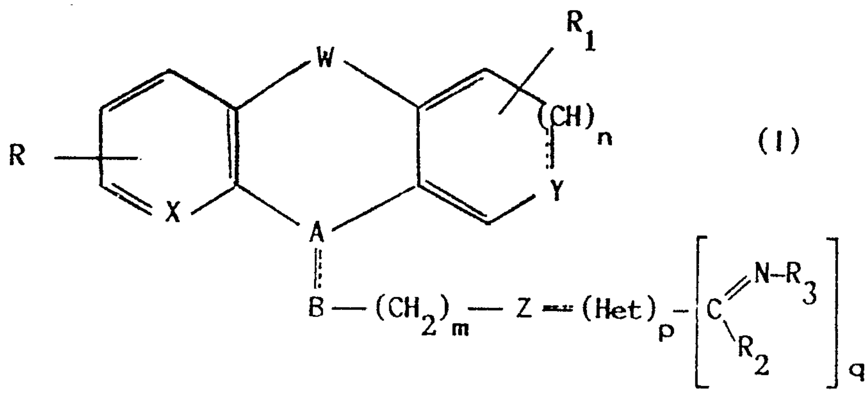

(I)

wherein the different substituents are as defined in claim 1, characterized in that, when q is 1 and $R_2$ is an amino group optionally substituted by a linear or branched $C_{1-4}$ alkyl or phenyl group, a compound of general formula (II)

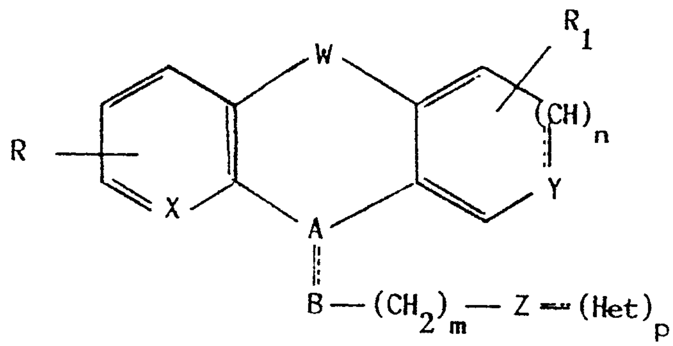

(II)

wherein the different substituents are as above defined, and provided that, when p is 1, Het is different from tetrahydropirimidine and contains at least one secondary amino function, is reacted, in the form of its addition salt with a mineral acid of formula HM, with cyanamide, optionally in the presence of a protic solvent at temperatures between 50° and 160° C, or with a reactive compound of formula (III)

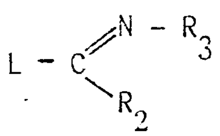

(III)

wherein L is a leaving group, $R_2$ and $R_3$ are as above defined, in a polar solvent at temperatures between 20° and 100° C.

8. Process for the preparation of compounds of formula (I) as defined in claim 7, characterized in that a nitroguanidine derivative of general formula (IV)

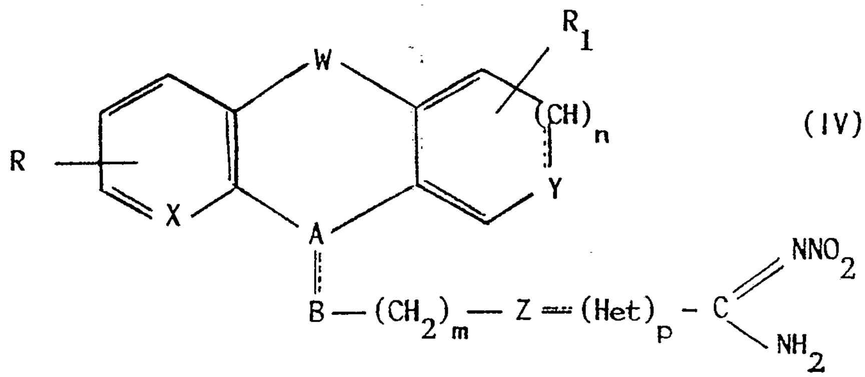

(IV)

provided that the nitroguanyl moiety is linked to the secondary nitrogen atom of Het, when p is 1, is reduced by hydrogen or hydrogen donor in the presence of a suitable catalyst and optionally of a suitable solvent at temperatures between 10° and 100° C.

9. Process for the preparation of compounds of general formula (I), as defined in claim 7, characterized in that a compound of general formula (VI)

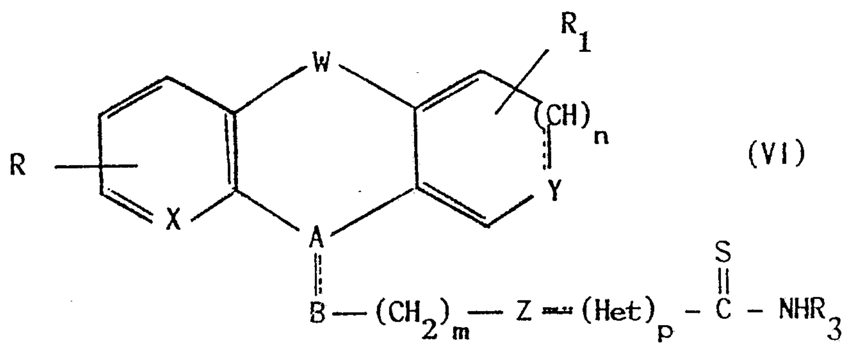

(VI)

provided that the thiocarbamoyl moiety is linked to the secondary nitrogen atom of Het, when p is 1, is reacted with an alkylating agent, and the corresponding isothiouronium intermediate is reacted in situ with an amine of formula $R_2$ - H, in which $R_2$ is as above dedefined, in the presence of a polar solvent at temperatures between 20° and 100° C.

10. Process for the preparation of compounds of general formula (I) as defined in claim 1, characterized in that, when q is 1 and $R_2$ is hydrogen atom or a $C_{1-4}$ alkyl, a compound of formula (II) as defined in claim 7, provided that, when p is 1, Het is different from tetrahydropyrimidine and contains at least one secondary amino function, is reacted with an addition salt of a compound of formula (VIII)

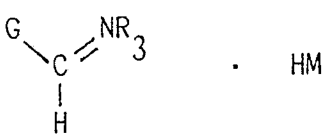

(VIII)

wherein G is a leaving group, when $R_2$ is hydrogen atom, or with a compound of general formula (IX)

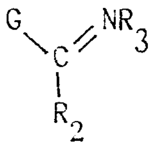

(IX)

wherein G, $R_2$ and $R_3$ are as above defined, when $R_2$ is $C_{1-4}$ alkyl in the presence of a polar solvent at temperatures between 10° and 70° C.

11. Process for the preparation of compounds of general formula (I) as defined in claim 1, characterized in that, when $R_2$ is hydrogen, a compound of formula (VI) is desulphurized with Raney-Nickel or $H_2O_2$ in an appropriate solvent at temperatures between 10° and 70° C.

12. Process for the preparation of compounds ot general formula (I) as defined in claim 1, characterized in that, when q is 1 and $R_2$ is an amino group optionally substituted by a linear or branched $C_{1-4}$ alkyl or phenyl group, a cyano derivative of general formula (X)

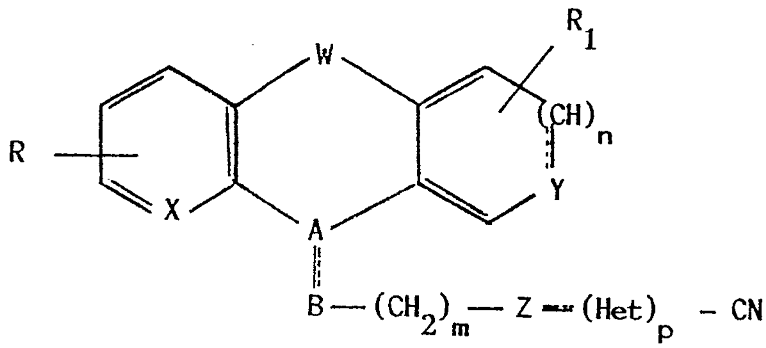

(X)

provided that the cyano moiety is linked to a carbon atom of Het when p is 1, and Het is different from tetrahydropyrimidine, is reacted with a substituted or unsubstituted ammonium salt of formula (XI)

$R_2H \bullet HM$ (XI)

wherein $R_2$ and HM are as above defined, or with a substituted or unsubstituted thiourea of formula (XII)

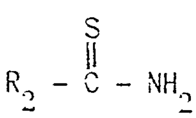

(XII)

at temperatures between 50° and 200° C.

13. Process for the preparation of compounds of general formula (I) as defined in claim 12, characterized in that a compound of formula (XI), as above defined, is reacted, as free base, with an imidate or an imidoyl derivative of general formula (XIII)

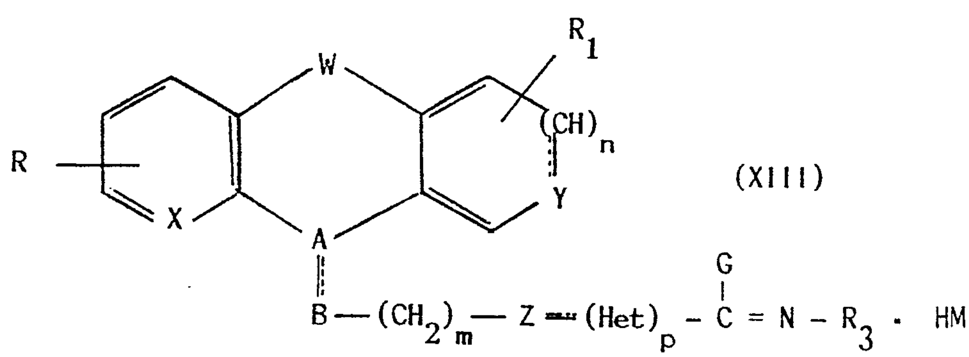

(XIII)

in the presence of a solvent at temperatures between 0° and 80° C.

14. Process for the preparation of compounds of general formula (I) as defined in claim 1, characterized in that, when m is 2, Het is a N-linked tetrahydropyrimidinyl group optionally substituted by $C_{1-4}$ alkyl or by an amino group, Z is absent and q is 0, a compound of general formula (XIV)

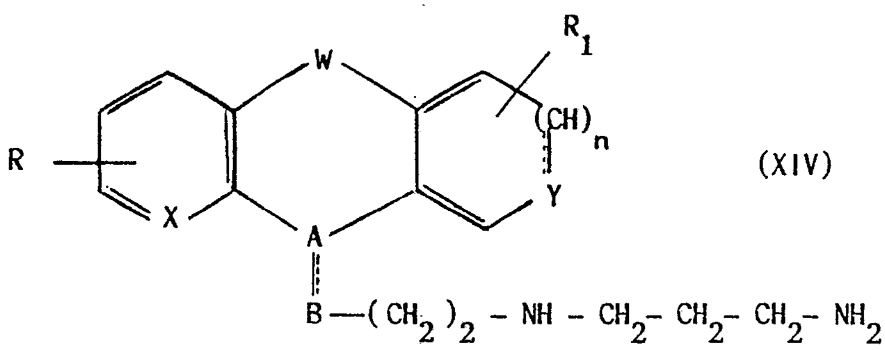

(XIV)

is reacted with a compound of general formula (XV)

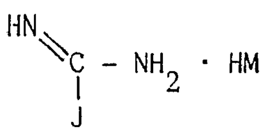

(XV)

wherein J is hydrogen atom, $C_{1-4}$ alkyl or an amino group, in the presence of a suitable solvent at temperatures between 0° and 80° C.

15. Process for the preparation of compounds of general formula (I) as defined in claim 14, characterized in that, when Het is N-linked tetrahydropyrimidinyl group substituted by an amino group, a compound of general formula (XVI)

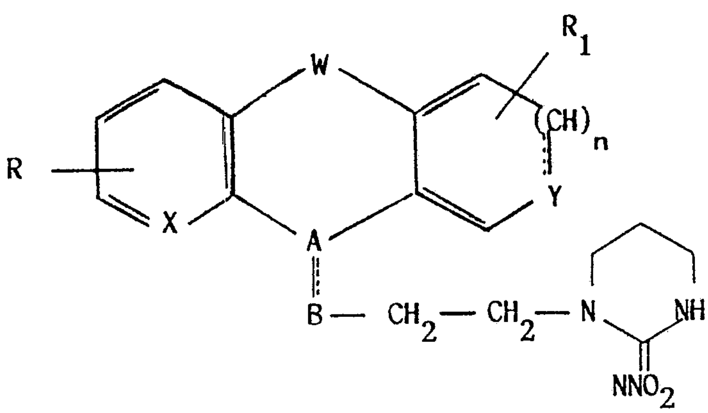

(XVI)

is reduced with a hydrogen or a hydrogen donor in the presence of a suitable catalyst and optionally in the presence of a suitable solvent at temperatures between 10° and 100° C.

16. Process for the preparation of compounds of general formula (I) according to claim 7, characterized in that the leaving group is 3,5-dimethylpyrazol-1-yl, lower thioalkyl or sulphonic group.

17. Process for the preparation of compounds of general formula (I) according to claim 8, characterized in that in the reduction of compounds of formula (IV) the hydrogen donor is formic acid, acetic acid, ammonium formate, cyclohexene or cyclohexadiene, and the solvent is formic acid, methanol or ethanol.

18. Process for the preparation of compounds of general formula (I) according to claim 10, characterized in that the leaving group is halogen, lower alkoxy, phenoxy or dichlorophosphoryl.

19. Process for the preparation of compounds of formula (I) according to claim 15, characterized in that

in the reduction of compounds of formula (XVI) the hydrogen donor is formic acid, acetic acid, ammonium formate, cyclohexene or cyclohexadiene, and the solvent is formic acid, water, methanol or ethanol.